# EUROPEAN PATENT SPECIFICATION

(11) **EP 3 305 290 B1**
(45) Date of publication and mention of the grant of the patent: **30.11.2022**
(21) Application number: 17193511.7
(22) Date of filing: 27.09.2017
(51) Int. Cl.: A61K 31/357, A61P 31/12

(54) **SILVESTROL AND EPISILVESTROL FOR USE IN THE TREATMENT OF VIRAL INFECTIONS**
SILVESTROL UND EPISILVESTROL ZUR VERWENDUNG BEI DER BEHANDLUNG VON VIRALEN INFEKTIONEN
SILVESTROL ET ÉPISILVESTROL POUR LEUR UTILISATION DANS LE TRAITEMENT DES INFECTIONS VIRALES

(30) Priority: 06.10.2016 EP 16192542
(43) Date of publication of application: 11.04.2018
(73) Proprietor: PHILIPPS-UNIVERSITÄT MARBURG, 35037 Marburg (DE); Justus-Liebig-Universität Gießen, 35390 Gießen (DE)
(72) Inventor: Grünweller, Arnold, 35094 Lahntal-Goßfelden (DE); Hartmann, Roland K., 35041 Marburg (DE); Lange-Grünweller, Kerstin, 35094 Lahntal-Goßfelden (DE); Schulte, Falk, 35041 Marburg (DE); Becker, Stephan, 35037 Marburg (DE); Biedenkopf, Nadine, 35392 Gießen (DE); Ziebuhr, John, 35614 Aßlar (DE); Müller, Christin, 35753 Greifenstein (DE); Schlitzer, Martin, 35043 Marburg (DE)
(74) Representative: Stumpf, Peter

(56) References cited:
- WO-A1-2013/016658
- BIEDENKOPF NADINE ET AL: "The natural compound silvestrol is a potent inhibitor of Ebola virus replication", ANTIVIRAL RESEARCH, vol. 137, 11 November 2016 (2016-11-11), pages 76-81, XP029857177, ISSN: 0166-3542, DOI: 10.1016/J.ANTIVIRAL.2016.11.011

## Description

### Field of the invention

The invention relates to the use of Silvestrol, Episilvestrol for treating infections with viruses whose protein translation requires a cap-structure at the 5'-end of viral mRNAs, especially for treating infections with (-)RNA-strand viruses with a negative-sense single-stranded RNA genome like the family of *Filoviridae* (Ebola- and Marburgvirus) as well as (+)RNA-strand viruses with a positive-sense single-stranded RNA genome like the family of *Coronaviridae* (e.g. MERS-CoV and SARS-CoV).

### Background of the technology

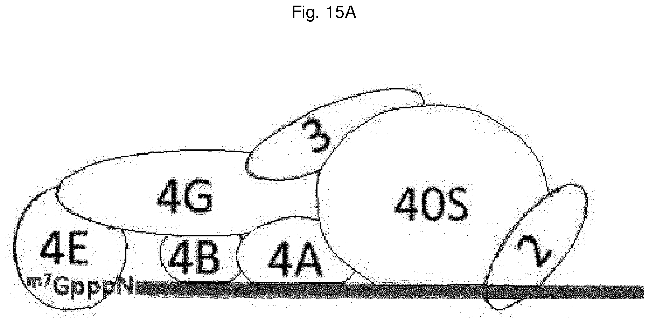

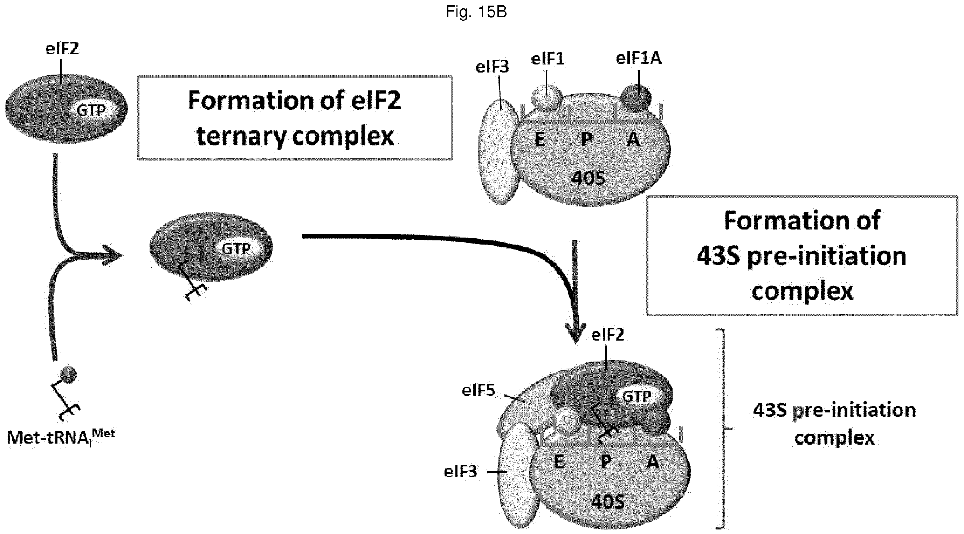

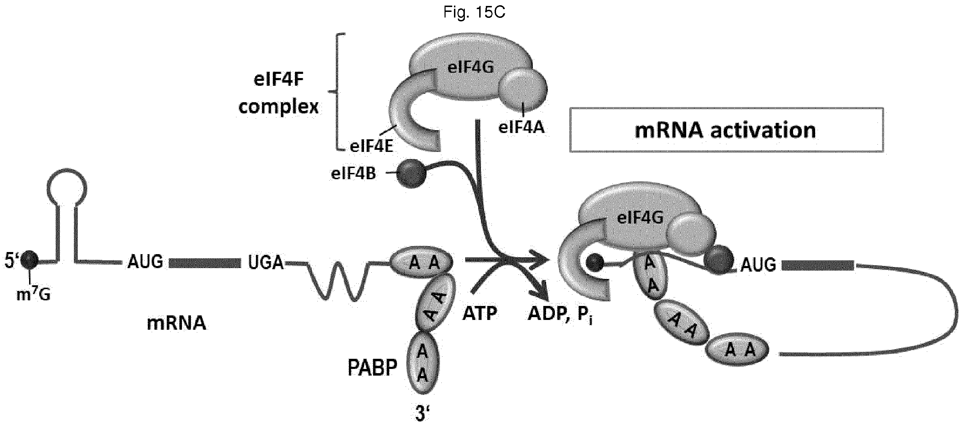

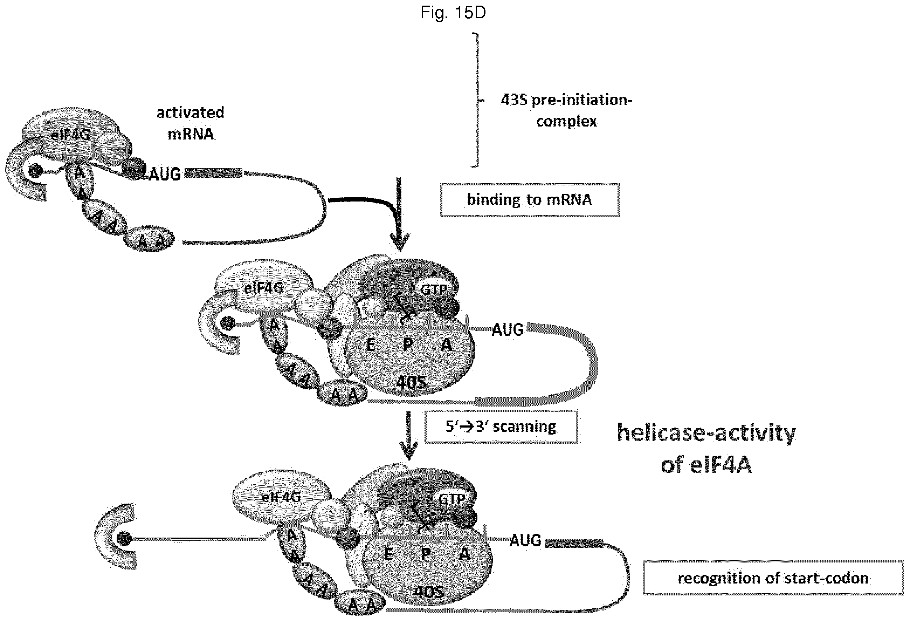

Silvestrol, a natural compound isolated from the plant *Aglaia foveolata,* belongs to the flavaglines, which have a cyclopenta[b]benzofuran skeleton in common. Flavaglines have cytostatic effects, however silvestrol and episilvestrol with an additional complex dioxanyl ring are the only natural flavaglines with cytotoxic effects. Silvestrol is a potent and specific inhibitor of the ATP-dependent DEAD-box RNA helicase eIF4A, which is part of the heterotrimeric translation initiation complex eIF4F in eukaryotes. The general mechanisms of viral translation initiation, relevant to the present invention are shown in Figs. 15 (A through D) and 16: The m7GpppN cap structure of RNA is specifically recognized by eIF4E, which recruits the cap-dependent translation initiation complex eIF4F, consisting of eIF4E, eIF4G and eIF4A, to the 5'-end of capped RNA. The different variants of cap-dependent translation initiation is more thoroughly displayed in Figs. 15B/C/D.

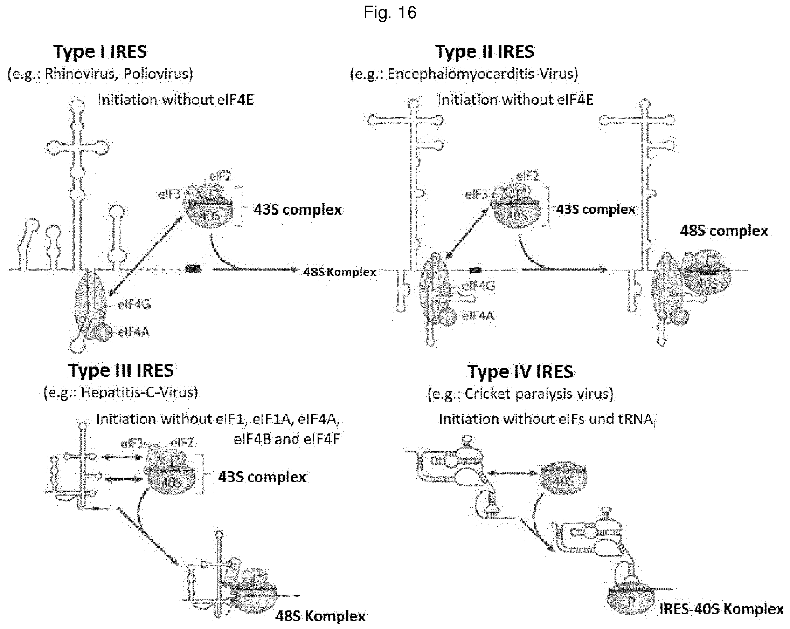

Conserved viral RNA structures located within the 5'-UTR of some viruses can directly promote translation initiation. These internal ribosome entry sites (IRES) are categorized into four different groups, each able to directly recruit a subset of initiation factors and the 40S ribosomal subunit in order to initiate translation (Fig. 16).

It has been proposed that Silvestrol increases the affinity of eIF4A for the bound target mRNA, thereby stalling the helicase on its RNA substrate. This might lead to depletion of eIF4A from eIF4F.

Silvestrol exerts potent antitumor activity *in vitro* and *in vivo* by inhibiting translation of short-lived oncoproteins, such as c-MYC and PIM1, whose mRNA 5'-UTRs are extended and include regions of stable RNA structures that require unwinding by eIF4A to create a binding platform for the 43S preinitiation complex.

WO2013/016658 A1 discloses the usage of silvestrol and episilvestrol for the treatment of cancer in combination with viruses as "viral vectors". The disclosed "viral vectors" are used as "vehicles" to transport genetic material resp. silvestrol or episilvestrol to the target cells (tumor cells). Thus WO2013/016658 A1 also only discloses treating cancer by administering silvestrol, episilvestrol and/or viral vectors, loaded with genetic material.

US2009/0082371 A1 teaches a method for treating cancer and viral infections of mammals by administering quinazoline derivatives, celastrol, cape, BAY 11-7082, rocaglamide and 7-Methoxy-5,11,12-trihydroxycoumestan to the patient. The usage of silvestrol and/or episilvestrol is not revealed or suggested.

Silvestrol is cytotoxic at low nanomolar concentrations to a large number of cancer cell lines and it is a very efficient anticancer agent in several tumor mouse models. Importantly, in non-cancer cells Silvestrol seems to be well tolerated and nontoxic up to low micromolar concentrations. The reasons are so far not well understood, however several (proto-)oncogenic mRNAs that are sensitive to Silvestrol harbor extended 5'-UTRs with extensive RNA secondary structure elements. It is therefore proposed that targeting eIF4A by Silvestrol might lead to mRNA discrimination during translation.

Highly structured 5'-UTRs are wide-spread among 5'-capped viral mRNAs, such as those transcribed from the (-)RNA-strand genome of Ebola virus (EBOV). In line with this, it is found herein that low nanomolar concentrations of Silvestrol strongly reduce EBOV titers in primary human macrophages by efficiently reducing viral protein expression without having cytotoxic side effects at concentrations of effective antiviral activity. Interestingly, the 5'-UTRs of the (+)RNA-strand genomes of coronaviruses MERS-CoV and HCoV-229E are also extended and contain stable RNA secondary structures. Therefore, coronaviruses are also sensitive towards Silvestrol treatment.

Negative- and positive-strand RNA ((-)RNA-strand; (+)RNA-strand) viruses are important human, animal and plant pathogens, which are defined by their single-stranded negative or positive-sense RNA genomes. The genomic RNAs of (+)RNA-strand viruses directly serve as templates for the synthesis of viral proteins in infected cells.

*Picornavirales* including rhinovirus A1 and poliovirus, characteristically translate their mRNAs in a 5'-cap-independent translation mechanism via an internal ribosomal entry site (IRES) sequence element within the 5' untranslated region (5'-UTR) of the viral genome. This allows translation by IRES-mediated recruitment of ribosomes to the mRNA 5'-UTRs. However, some IRES elements still require at least some cellular translation initiation factors, whereas others are able to intitiate translation entirely independent of these proteins.

Chu et al. (Chu et al. 2016, Cell Reports 15, 2340-2347) show that the antineoplastic activity of rocaglates is a consequence of the eIF4A1 inhibition and also found that a synthetic derivative is even more efficient than the natural compound. Therefore, eIF4A is discussed as a promising new drug target for cancer treatment.

The recent outbreak of the Ebola virus (EBOV) in West Africa caused more than 28,000 cases with at least 11,000 fatalities, revealing that effective viral inhibitors with potential broad-spectrum activity are urgently needed. EBOV is a negative-sense single-stranded RNA virus encoding seven genes. Transcription of the genes is accomplished by the viral polymerase complex resulting in monocistronic, 5' capped and 3' polyadenylated mRNAs. As for all viruses, EBOV protein synthesis depends on the cellular translation machinery. Interestingly, some EBOV mRNAs harbor long 5'-UTRs and all EBOV mRNAs were predicted or demonstrated to adopt stable RNA secondary structures in their 5'-UTRs. Thus efficient cap-dependent translation of EBOV mRNAs also requires the host helicase activity of eIF4A to unwind such structures during initiation of translation.

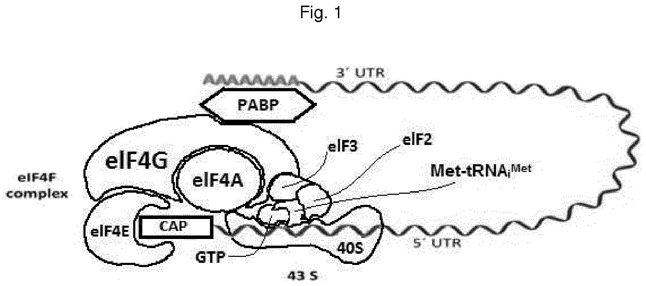

Silvestrol, as well as other rocaglates, exerts very low toxicity within normal cells and, due to its highly specific inhibition of the eIF4A RNA-helicase, a selective inhibition of the cap-dependent translation of proto-oncogenes (cf. Fig. 1).

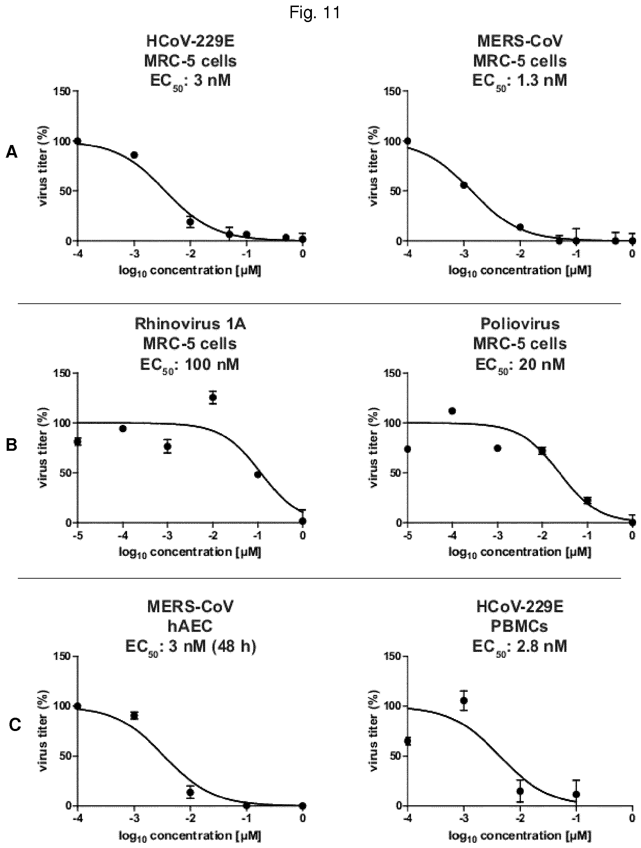

Thus it is known in the state of the art to use Silvestrol (a derivative of rocaglate with a cyclopenta[b]benzofuran skeleton) for the treatment of cancer. It is not known in the state of the art to use Silvestrol or Silvestrol analoga (as well as Episilvestrol) for the treatment of virus infections. Moreover, in comparison to other known compounds with antiviral activity, Silvestrol, Episilvestrol and their analoga exert a very potent antiviral activity against Coronaviruses with a (+) RNA strand genome whose protein translation is also cap-dependent and at the same time has no specific antiviral activity against viruses whose protein translation can be initiated by an internal ribosome entry site (IRES) (for example Rhinovirus 1A and Poliovirus). However, more recent data has shown that the poliovirus is sensitive towards Silvestrol treatment with an EC₅₀ value of 20 nM which is in line with the known eIF4A dependency of the Type I IRES found in the 5'-UTR of the poliovirus. The rhinovirus A1 also harbours a Type I IRES in its 5'-UTR but this virus is less sensitive towards Silvestrol treatment with an EC₅₀ value of 100 nM (Fig.11).

Silvestrol is generally inhibiting the cap-dependent translation of structured viral mRNAs. Therefore Silvestrol, Episilvestrol and their analogs and derivatives are effective as "broadband" virostatics. This is, because only if viruses can perform a cap-independent translation (e.g. Rhinoviruses and Polioviruses), there is no main dependency to cellular eIF4A-helicase, due to the fact that then the ribosomeas-sembly can take place directly at a IRES-sequence. Thus the unwinding of RNA secondary structures with the help of eIF4A in order to provide a binding-platform for the 43S preinitiation complex is not necessary in this case. It is well known to the person skilled in the art that this effect - so far found at the Ebola- and Coronaviruses (Coronaviridae like MERS-CoV) - may universally prevail with all types of viruses which translate cap-dependently, i.e. without IRES (e.g., Flaviviridae like the Zikavirus and the Denguevirus, Togaviridae like the Chikungunyavirus or the Semliki-Forest virus, Orthomyxoviridae like Influenza viruses-, Bunya-, Rhabdo-, Paramyxoviridae etc.). Thus it is obvious to the person skilled in the art that the scope of the invention is not confined to the Filoviridae (Ebola- and Marburgvirus), but comprises all types of viruses which also perform cap-dependent protein translation.

Surprisingly, further experiments have shown that Silvestrol has an even broader effective range than was previously considered according to the preceding paragraph:
The picornaviruses rhinovirus A1 and poliovirus, which use an IRES-driven cap-independent translation initiation mechanism, are less sensitive against Silvestrol treatment. Polioviruses, which require the activity of eIF4A, nevertheless respond to Silvestrol in MRC-5 cells with a calculated EC₅₀ value of 20 nM, which is about 10-fold less efficient compared to MERS-CoV and HCoV-229E with EC₅₀ values of 1.3 nM and 3 nM, respectively. The EC₅₀ value for Silvestrol against rhinovirus A1 is about 100 nM, indicating a less distinct dependency on eIF4A for its IRES-driven translation. For the highly pathogenic MERS-CoV, additional testing of antiviral activity in primary human alveolar epithelial cells (hAEC) as well as in PBMCs again shows very potent and non-toxic antiviral effects of Silvestrol. In line with this, Silvestrol strongly inhibits the expression of the CoV proteins N and nsp8 and the formation of viral replication/transcription complexes. The data reveal a broad-spectrum antiviral and non-toxic activity of Silvestrol by inhibiting eIF4A-dependent viral mRNA translation.

Because the inhibition of the cap-dependent translation is most effective during early states of viral infections, the application of Silvestrol, Episilvestrol and their derivatives and analogues is most helpful at acute infections with viruses that show short viral replication cycles.

The scope of the invention is even more obvious if one considers that most types of viruses can escape antiviral active principles that are aiming towards viral targets by use of so called escape-mutations. This strategy is very difficult with host cell targets that are also concerning vital processes for viral growth like the eIF4A-helicase, for any mutation of eIF4A will most likely also impair vital processes of virus proliferation.

Regarding Coronaviruses Silvestrol shows an extraordinary high efficacy with EC₅₀-values of 1-3 nM. Cell cytotoxicity is with CC₅₀-values of about 2 µM about 1000 times higher, leading to a selectivity index of about 1000. This is an extremely high value, showing that Silvestrol, Episilvestrol and their analogs and derivatives offer a very high antiviral specifity.

### Content of the invention

Herein the antiviral effects of Silvestrol on cap-dependent EBOV, MERS-CoV and the human HCov-229E in Huh-7 cells, primary macrophages, MRC-5 cells (human embryonic lung fibroblasts), hAEC (human alveolar epithelial cells) and PBMCs (peripheral blood mononuclear cells) are analyzed. In order to investigate the antiviral relevance of eIF4A inhibition by Silvestrol in more detail, also the effects of Silvestrol on rhinovirus A1- and poliovirus-infected cells are analyzed, because it is known that picornaviruses commonly require the action of a specific set of translation initiation factors such as eIF4A, for initiating IRES-dependent translation.

In the following, the basic experiments are first revealed, together with the conclusions resulting from them regarding the scope of the invention. Secondly the most recently performed experiments are revealed, leading to a broader scope of the invention than originally visible from the basic experiments.

### Silvestrol inhibits EBOV propagation in Huh-7 cells

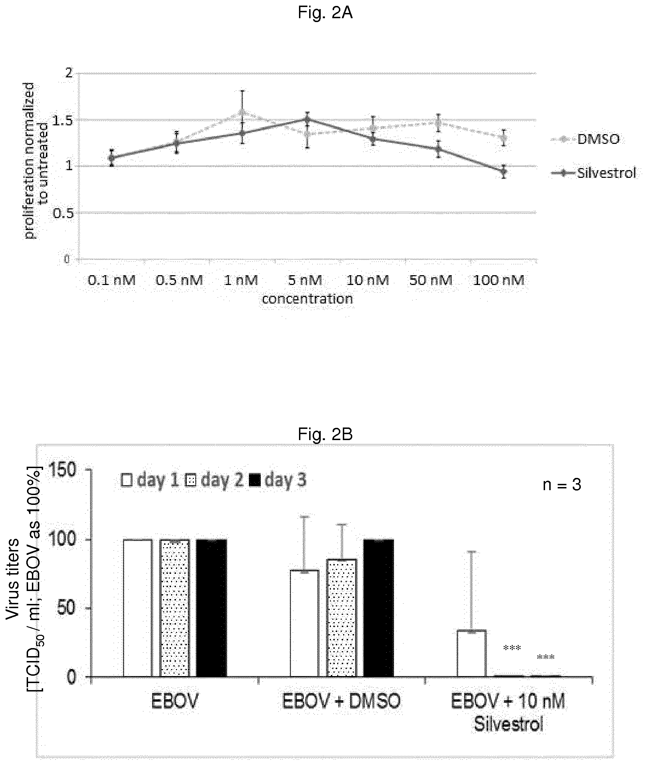

The effects of Silvestrol on proliferation of Huh-7 cells, a standard hepatoma cell line to investigate EBOV is analyzed by using Silvestrol concentrations in a range between 0.1 nM - 100 nM. Up to 10 nM Silvestrol no obvious effects on proliferation could be detected. At higher concentrations cell proliferation is weakly affected compared to the DMSO control (Fig. 2A).

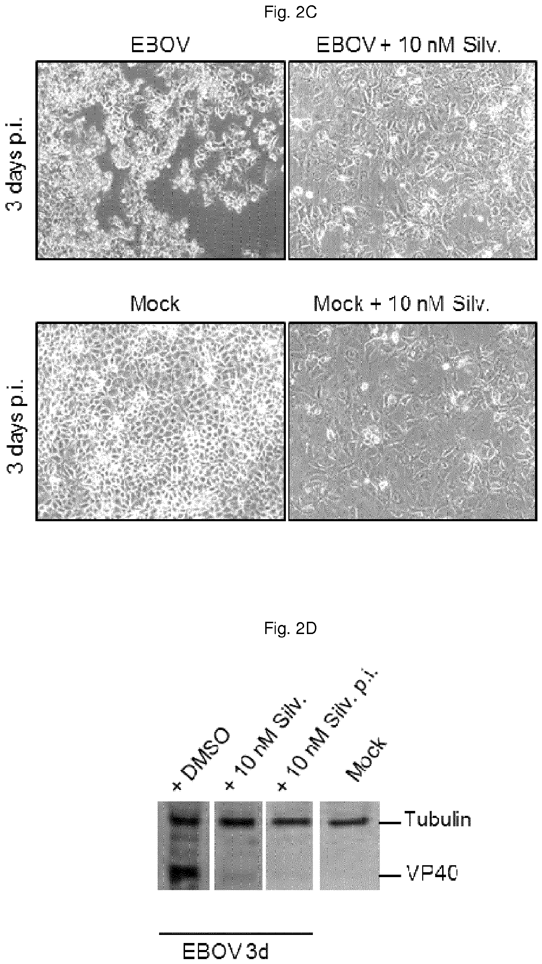

For testing the potential antiviral activity of Silvestrol, Huh-7 cells are preincubated for 2 h with 10 nM Silvestrol and then infected with EBOV at a multiplicity of infection (MOI) of 0.1. Viral titers of supernatants were determined by TCID₅₀ analysis in VeroE6 cells at day 1, 2, and 3 (p.i.). Remarkably, a concentration of 10 nM Silvestrol substantially inhibits EBOV infection (Fig. 2B) without changing cell morphology as determined by light microscopy (Fig. 2C).

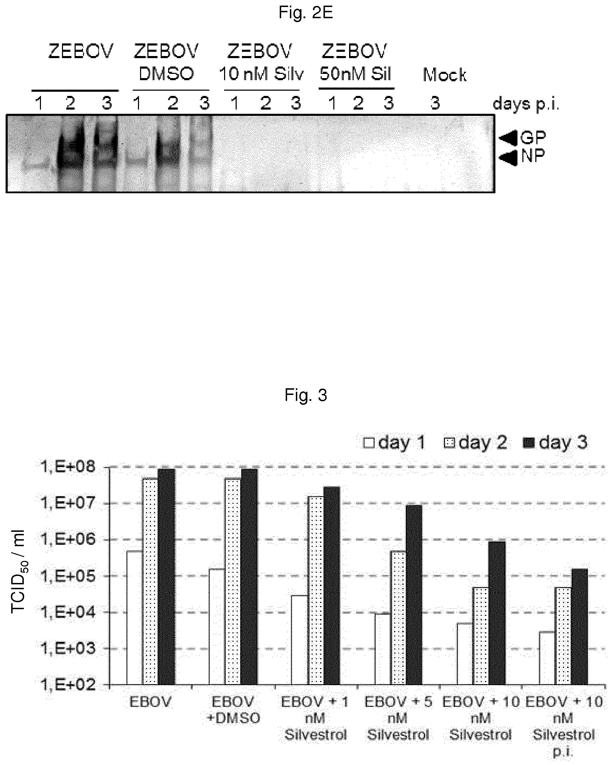

The specificity of Silvestrol on viral mRNA translation is shown via investigation of its impact on expression of the EBOV protein VP40 and the housekeeping protein tubulin by Westernblotting. A strong reduction of VP40 levels by treating cells with 10 nM Silvestrol 2 h before or post EBOV infection is taking place (Fig. 2D). Similar reduction in protein levels also does appear for the EBOV proteins NP and GP (Fig. 2E). These results clearly show a substantial antiviral effect of Silvestrol in EBOV-infected Huh-7 cells. It is obvious to a person skilled in the art that analogs and derivatives of Silvestrol and Episilvestrol may offer the same or even more intense antiviral effects and/or the same or even less cytotoxicity. Therefore the scope of the invention is not limited to Silvestrol, but also comprises analogs and derivatives of Silvestrol and Episilvestrol.

At a concentration of only 1 nM Silvestrol causes a reduction of the EBOV titer by almost one order of magnitude and at 5 nM the inhibitory effect of Silvestrol increases up to 1-2 log orders (Fig. 3). Again, treatment of Huh-7 cells with 10 nM Silvestrol inhibits viral propagation efficiently. A comparable antiviral effect can also be measured when Silvestrol treatment is started immediately after EBOV infection (p.i.) (Fig. 3, last panel).

Previous experiments suggested that Silvestrol is not efficient against Rhinovirus 1A harbouring an IRES, but additional experiments now surprisingly show that Silvestrol has a much broader efficacy against Rhinovirus 1A and also against poliovirus, representing different species in the familiy *Picornaviridae.*

Silvestrol can also inhibit the propagation of ssRNA viruses with a (+) RNA genome. The effects of Silvestrol in primary MRC-5 cells (derived from normal lung tissue) infected with human pathogenic Coronaviruses proves this.

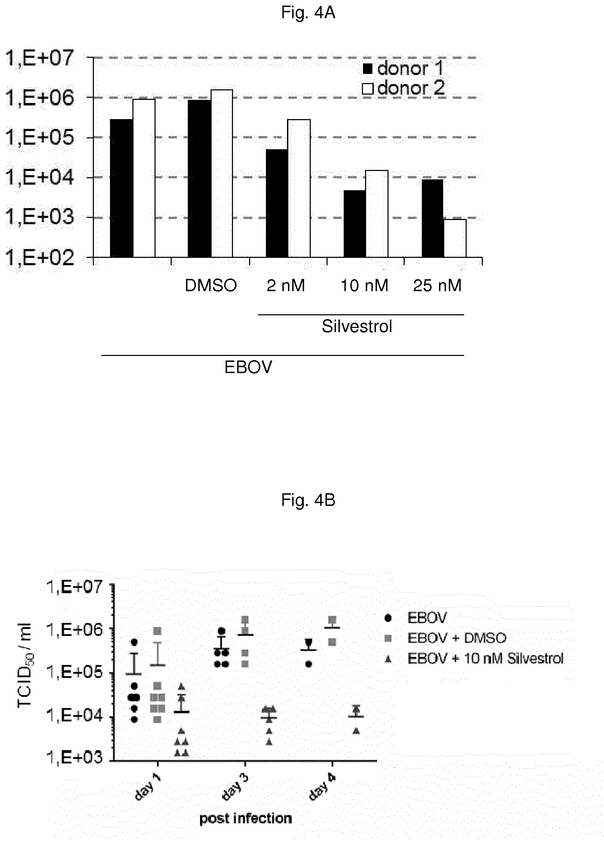

Beneath Cap-dependent translation several viruses, like Picornaviruses, can initiate translation via an IRES. Therefore the antiviral activity of Silvestrol in Rhinovirus 1A infected Hela cells was investigated, finding an EC₅₀ value of 400 nM, which is about 10-fold higher compared to Huh-7 infected HCoV-229E cells (EC₅₀ of 40 nM; see Fig. 4A-D; Fig. 4A-D shows the first data gathered regarding this context, testing the activity of Silvestrol against rhinovirus A1 with Hela-cells; additional investigations using primary MRC-5 cells surprisingly show that picornaviruses are also sensitive towards Silvestrol and/or Episilvestrol). Since Silvestrol has potent antitumor activity in a large set of cancer cell lines, the observed reduction in virus titer in Rhinovirus 1A infected Hela cells at concentrations >100 nM should be mainly caused by antiproliferative effects of Silvestrol through the inhibition of proto-oncogenes and by induction of apoptosis. From this it is to be concluded that Silvestrol is mainly active against viruses with Cap-dependent translation initiation.

This finding is further substantiated by the effect of Silvestrol in another Picornavirus, namely Polioviruses, which also initiate translation via an IRES. Again, the effect of Silvestrol in Poliovirus infected VeroE6 cells is taking place with an EC₅₀ value of about 100 nM, a concentration where unwanted effects of Silvestrol against proto-oncogenes are expected. Moreover, more recently performed experiments, also using primary MRC-5 cells, surprisingly showed that Silvestrol and/or Episilvestrol do indeed also have a remarkable antiviral effect against the poliovirus.

### Antiviral activity of Silvestrol on EBOV-infected human primary macrophages

Primary human macrophages, which are one of the first target cells during EBOV infections, are a highly relevant cellular system to thoroughly investigate the antiviral effects of Silvestrol on EBOV replication. Using the above mentioned preincubation setup, the impact of Silvestrol on EBOV infection of macrophages isolated from two different donors at 2, 10 and 25 nM is analyzed. A concentration-dependent inhibition of viral titers between 1 to 3 log orders can be observed (Fig. 4A). Repetition of the experiment with macrophages isolated from more donors verifies the antiviral effect of Silvestrol: Preincubation of EBOV-infected macrophages from seven donors with 10 nM Silvestrol confirmes the reduction of viral titers by up to two orders of magnitude (Fig. 4B).

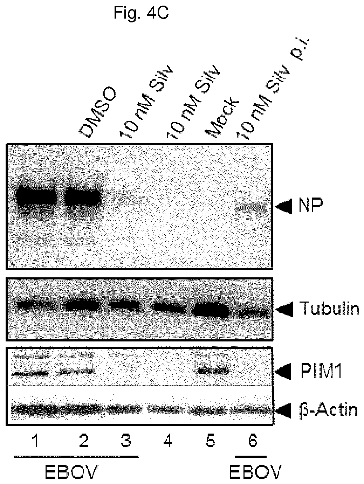

Upon analyzing the expression level of the EBOV NP protein by Westernblotting a potent downregulation is observed (Fig. 4C, example of one donor, lane 3). The same is true when Silvestrol is administered after EBOV infection (Fig. 4C, lane 6). As mentioned above, Silvestrol inhibits translation of highly structured 5'-UTRs of proto-oncogenes and thus leaves the expression of housekeeping genes largely unaffected. Therefore, the impact of Silvestrol on the levels of the proto-oncogenic kinase PIM1 in primary macrophages is tested, using β-Actin as a housekeeping control protein. As expected, PIM1 levels are strongly decreased (to almost below detection limits) in the presence of 10 nM Silvestrol (Fig. 4C).

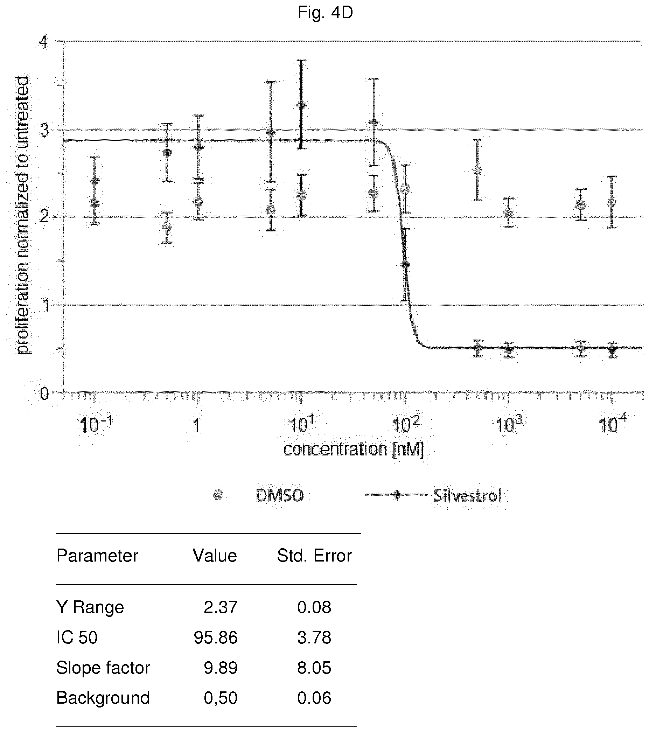

The toxicity of Silvestrol is quantified in primary macrophages using the WST-1 viability assay in a concentration range between 0.1 nM - 10 µM. This reveales an IC₅₀ value of about 96 nM, with essentially no sign of toxicity up to at least 50 nM Silvestrol (Fig. 4D). In accordance with this result it is known in the state of the art that Silvestrol exerts no toxicity at therapeutic doses in antitumorigenic animal studies. The results revealed herein prove the existence of a therapeutic window from below 1 nM until more than 1 µM within which Silvestrol can be applied to efficiently inhibit EBOV propagation at non-toxic concentrations by affecting cap-dependent translation initiation of viral mRNAs.

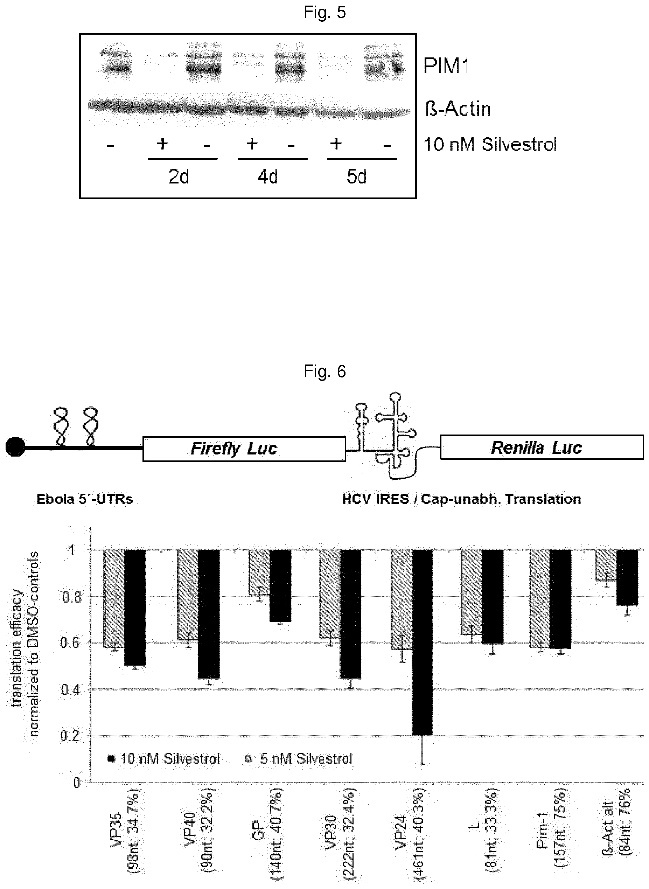

Importantly, this inhibitory effect (cf. Fig. 4C) continued for at least 5 days using a single dose of Silvestrol, without affecting cellular β-Actin levels (Fig. 5).

Fig. 6 shows results of experiments regarding the analysis of the 5'-UTRs of Ebola mRNAs by using a dual luciferase reporter gene assay. The 5'-UTRs of the EBOV genes VP35, VP40, GP, VP30, VP24 and L were cloned infront of the firefly luciferase gene to determine the effects of Silvestrol treatment on firefly luciferase expression. Additionally the reporter vector contain an IRES-driven renilla luciferase gene to quantify cap-independent translation for normalization of the plasmid transfection rate. Treating transfected cells with Silvestrol has no obvious effect on renilla luciferase activity. The effects of Silvestrol on firefly luciferase activity were concentration dependent with a maximum inhibitory effect of 80% in case of the plasmid that harbors the VP24 5'-UTR infront of the firefly luciferase reporter gene. The 5'-UTR of PIM1 was used as a positive control and the 5'-UTR of β-Actin as a negative control.

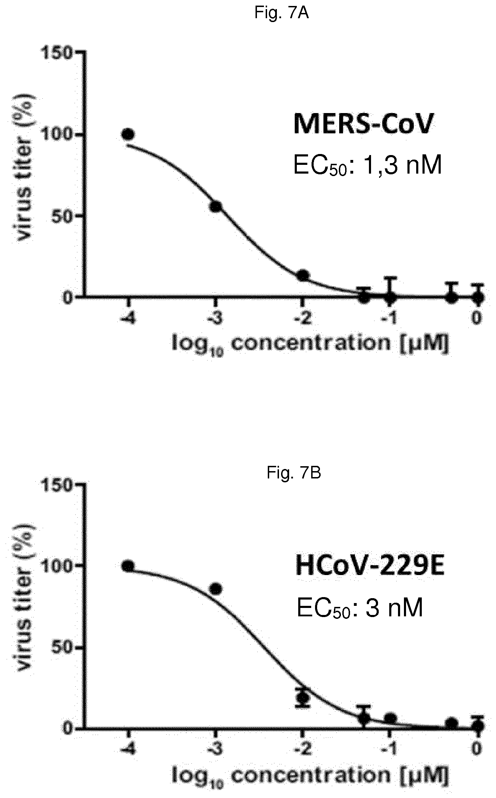

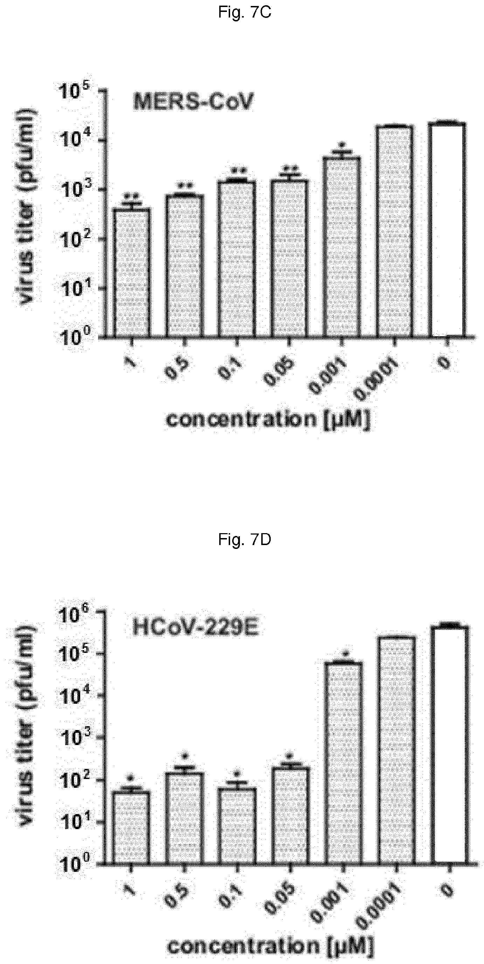

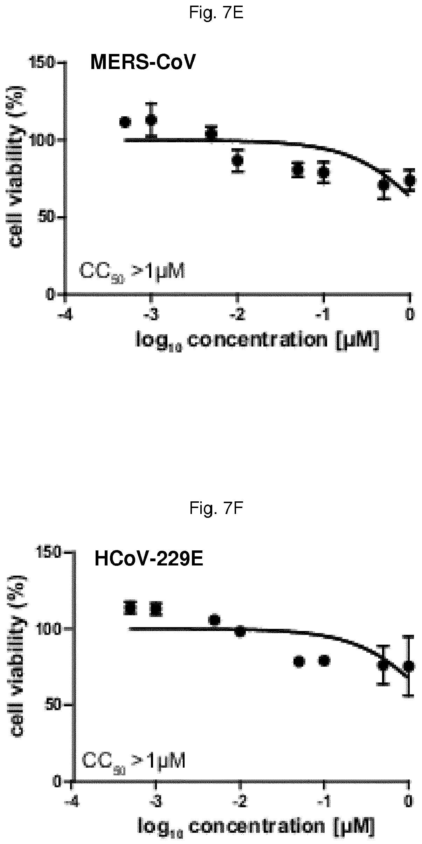

Silvestrol is also inhibiting the MERS-CoV (high pathogenic) and HCoV-229E (low pathogenic) replication in primary MRC-5 lung-cells, as can be seen on Fig. 7A/B/C/D. The viral titers of infected cells were determined 24 h post infection and EC₅₀-values were determined. The cell toxicity of Silvestrol in primary MRC-5 cells is shown in Fig. 7E/F (type MERS-CoV (highly pathogenic) and HCoV-229E (low pathogenic)) by analyzing the cell viability 24 h post infection. The data presented in Fig. 7A-F shows that:
- The cell toxicity of Silvestrol in MRC-5 cells is above 1 µM
- EC₅₀ of Silvestrol within cells of MERS-CoV type is about 1.3 nM
- EC₅₀ of Silvestrol within cells of HCoV-229E type is about 3 nM
- The selectivity index (CC₅₀:EC₅₀) is about 500 - 1000.

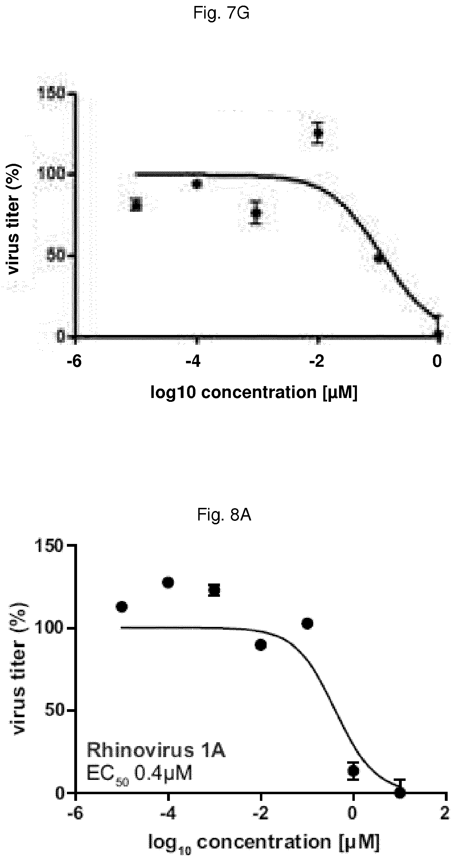

Regarding the IRES-dependent translation on cell line MRC5, infected with Rhinovirus 1A, Fig. 7G shows that Silvestrol offers an EC₅₀-value of 0.1 µM and a CC₅₀-value of 1.7 µM. Regarding the Poliovirus a CC₅₀-value of 2 µM is observed.

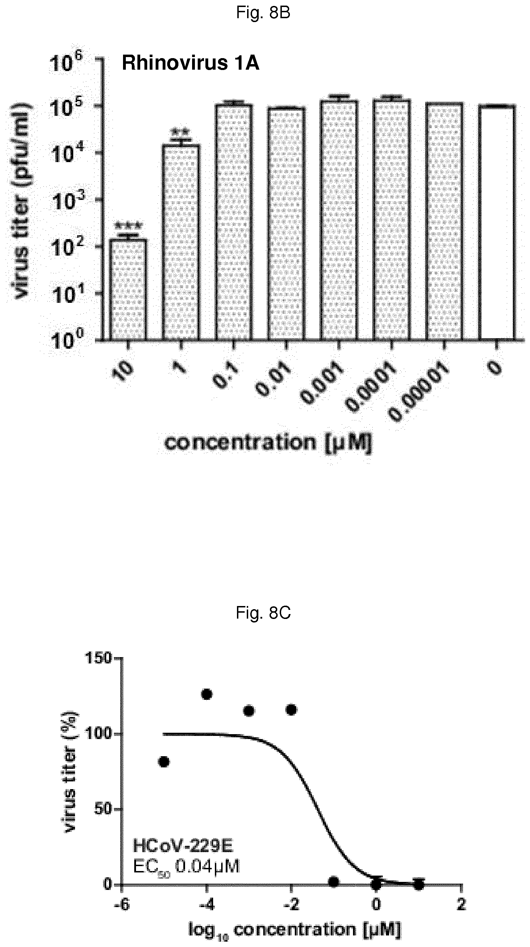

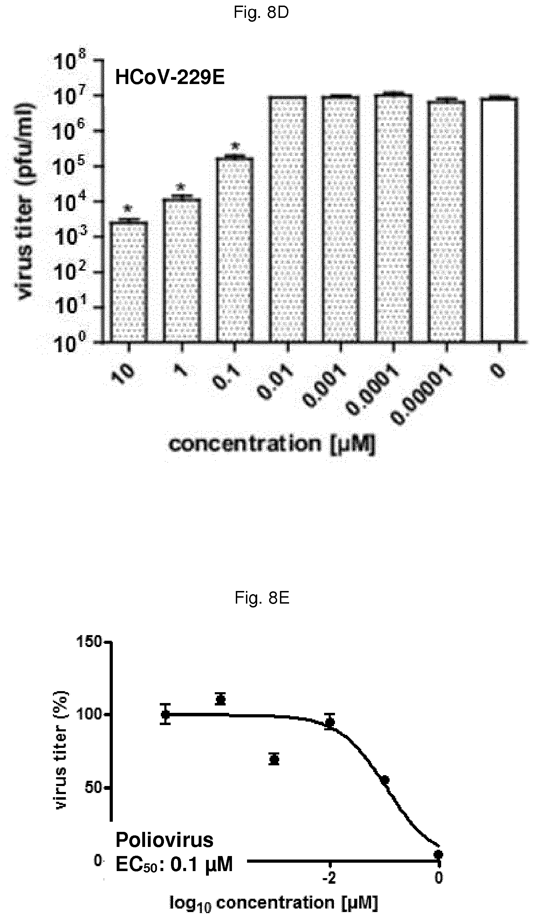

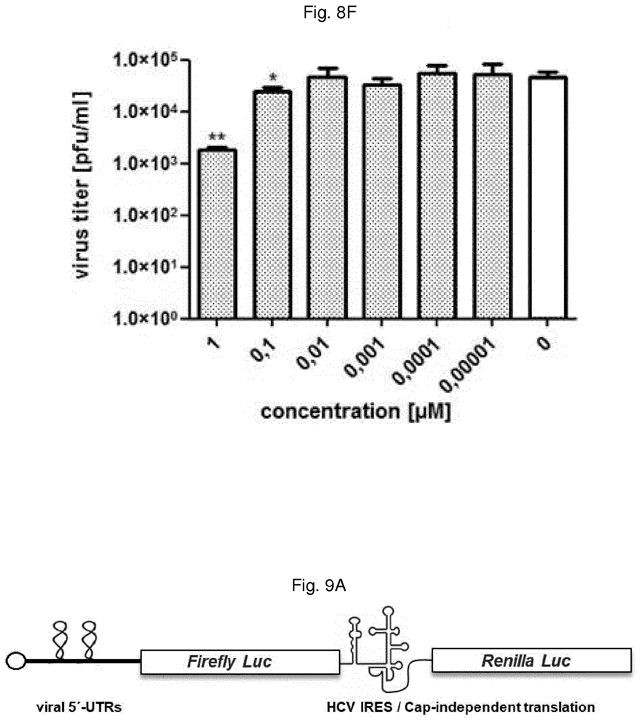

That there is - under certain circumstances - no specific inhibition by Silvestrol of the translation of IRES-dependent viruses (Picornaviruses ((+) ssRNA), e.g. Rhinovirus 1A, Poliovirus) can be seen on Fig. 8A/B/C/D/E/F, based on experimental data gathered by use of tumor cell lines. The data presented in Fig. 8A-F shows that:
- With Rhinovirus 1A (Picornavirus) the EC₅₀-value is about 400 nM.
- With Poliovirus (Picornavirus) the EC₅₀-value is about 100 nM.
- At concentrations of more than 50 nM Silvestrol within tumor-cells anti pro-literative effects are generated via inhibition of proto-oncogenes.

It is well known in the state of the art that tumor cells are quite sensitive against Silvestrol and/or Episilvestrol at concentrations of about 10 nM. The cytotoxicity of Silvestrol and Episilvestrol towards primary cells is remarkably lower, so that toxic effects in primary cells are appearing at much higher concentrations of about 0.5 to 2 µM.

Now the most recently performed experiments are revealed, leading to an even broader scope of the invention than described above:

### Effects of Silvestrol on translation of a luciferase reporter mRNA fused to viral 5'-UTRs

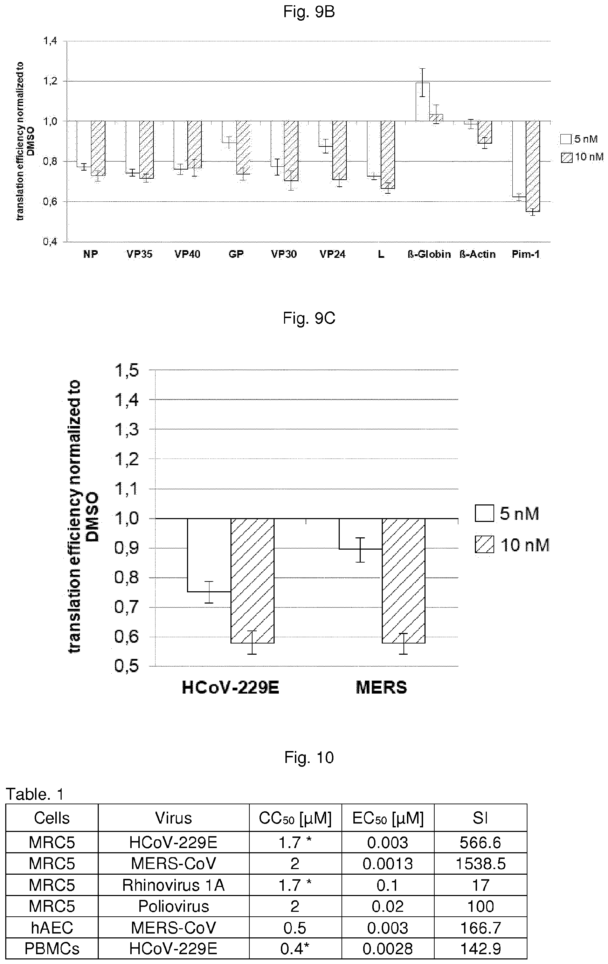

As described above Silvestrol is an efficient translational inhibitor of several EBOV mRNAs and of the proto-oncogenic PIM1 kinase mRNA. In order to more thoroughly understand the effects of Silvestrol on cap-dependent or IRES driven viral protein synthesis, a dual luciferase reporter assay was performed. The 5'-UTRs of all seven EBOV mRNAs were cloned upstream of a firefly luciferase gene. After transcription the corresponding mRNAs are 5'capped in the host cell for efficient translation. For normalization, the reporter plasmid-encoded mRNA contains a second reading frame under control of an HCV IRES to express a Renilla luciferase (Fig. 9A). Importantly, HCV has a Type III IRES, which is independent from eIF4A and other translation initiation factor activities, thus the eIF4A-selective inhibitor Silvestrol cannot inhibit Renilla luciferase translation. As a positive control to determine the inhibitory potency on translation by Silvestrol, the 5'-UTR of PIM1 is used because cap-dependent translation of this oncogenic kinase is known to require the activity of the eIF4A helicase. In all cases, dose-dependent inhibition of firefly luciferase activity by 23-33 % is observed upon exposure of cells to Silvestrol concentrations of 5 or 10 nM (cf. Fig. 9B). As negative controls, the unstructured and short 5'-UTR of β-globin mRNA is applied as well as the 5'-UTR of β-actin mRNA. In both cases no significant reduction of firefly luciferase activity could be observed by Silvestrol treatment (Fig. 9B). From these results it is concluded - without commitment to a certain theory - that the dual luciferase assay is a suitable system to analyze the Silvestrol sensitivity of 5'capped viral mRNAs that harbor stable RNA secondary structures in their 5'-UTRs.

Also, the Silvestrol responsiveness towards the highly structured 5'-UTRs from the human coronavirus HCoV-229E and the highly pathogenic MERS-CoV has been investigated. In both cases, a relatively strong decrease of luciferase activity in the same range as for the PIM1 positive control (∼40%) is measured at a silvestrol concentration of 10 nM (Fig. 9C), indicating that also mRNAs of (+) ssRNA viruses depend on eIF4A and may thus be suitable targets to silvestrol treatment.

The new results revealed herein surprisingly show that the antiviral activity of Silvestrol is thus not restricted to EBOV. Other RNA viruses e.g. coronaviruses with cap-dependent translation and structured 5'-UTRs are also sensitive to Silvestrol treatment.

### Effects of Silvestrol on viruses with 5'-capped or IRES containing mRNAs

The studies with EBOV already showed specific and potent antiviral activity of Silvestrol in EBOV-infected primary human macrophages. These results are substantiated by further analyzing the effects of Silvestrol on viral titers after infection with CoV or Picornaviruses by determining EC₅₀ and CC₅₀ values in human primary lung fibroblasts (MRC-5 cells). Cells are infected with HCoV-229E, MERS-CoV, RVA1 or PV with an MOI of 0.1 and Silvestrol is added to the infected cells in a concentration range from 0.1 nM to 1 µM. Again, viral titers are determined 24 h p. i. (post infection). The obtained EC₅₀ values in HCoV-229E and MERS-CoV infected cells are calculated as 3 nM and 1.3 nM, respectively (Fig. 11). Cytotoxicity of Silvestrol after 24 h in MRC-5 cells is determined at 33 °C (corresponding to HCoV-229E and RVA1 infection) and at 37 °C (corresponding to MERS-CoV and PV infection) which are the optimal temperatures for infection by the respective viruses. However, similar CC₅₀ values are determined at the two temperatures, about 1.7 µM at 33 °C and 2 µM at 37 °C (see Table 1, Fig. 10 and Fig.11). The resulting selectivity indices (CC₅₀/EC₅₀) are 566 for HCov-229E and 1538 for MERS-CoV, demonstrating very low toxicity and very potent antiviral activity of Silvestrol in primary cells (Table 1, Fig. 10). The EC₅₀ values for RVA1 and PV infected MRC-5 cells are about 100 nM and 20 nM, respectively, demonstrating remarkable antiviral activity of Silvestrol in primary MRC-5 cells also when eIF4A-dependent IRES-driven translation is used by the virus. However, RVA1 is about 100-fold and PV about 10-fold less sensitive to Silvestrol treatment compared to the analyzed coronaviruses (see Table 1, Fig. 10 and Fig. 11), thus pointing to a less prominent role of eIF4A especially during RVA1 translation initiation. So far, potent antiviral activity of Silvestrol is observed in primary cells against coronaviruses and the Ebola virus. The Type I IRES-containing RV A1 and PV are also sensitive to Silvestrol treatment in primary cells, thus showing a -broad-spectrum antiviral activity of Silvestrol against viruses with 5'-capped mRNAs and structured 5'-UTRs as well as IRES-containing viruses whose translation initiation depends on eIF4A helicase activity.

The studies have been extended to the highly pathogenic MERS-CoV by analyzing the antiviral effects of Silvestrol in infected human alveolar epithelial cells (hAEC). These cells are isolated directly from a patient according to the ethical guidelines. Again, hAEC is infected with MERS-CoV at a MOI of 3 and Silvestrol is added in a concentration range from 0.1 nM to 1 µM. The viral titers are determined 48 h p.i. and an EC₅₀ value of 3 nM is derived (Fig. 11), which is comparable to the EC₅₀ value obtained with MRC-5 cells (see above). A CC₅₀ value of 500 nM is determined for Silvestrol in hAEC 48 h p.i., corresponding to to a selectivity index of 166 (Table 1, Fig. 10).

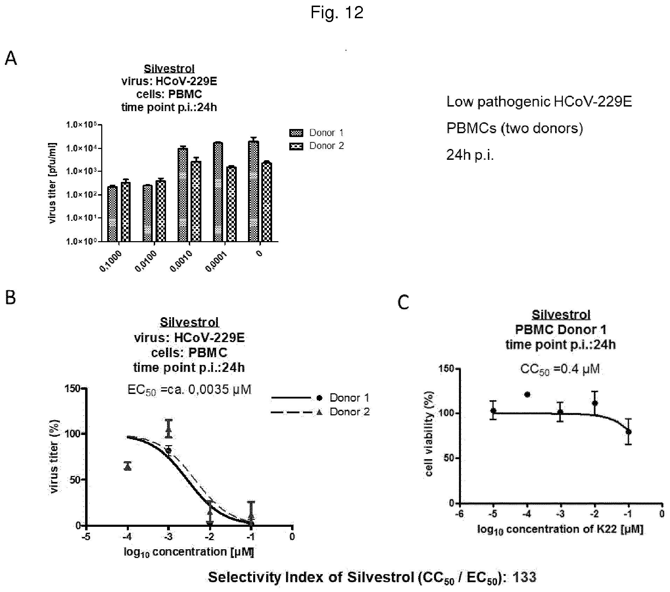

Peripheral blood mononuclear cells (PBMC) are important primary target cells for several viral infections. Therefore, the effects of Silvestrol in PBMCs, isolated from a human donor and infected with HCoV-229E, have been analyzed as well. The measured CC₅₀ value of 400 nM and the EC₅₀ value of 2.8 nM correspond to a selectivity index of about 143 (Fig. 11 and Table 1, Fig. 10). A similar result was obtained for a second donor yielding an EC₅₀ value of about 3.5 nM (Fig. 12). These experiments show that the potent and non-toxic antiviral activities of Silvestrol against MERS-CoV and HCoV-229E are not cell type-specific.

### Silvestrol inhibits formation of replication/transcription complexes, viral RNA synthesis and the translation of HCoV-229E proteins

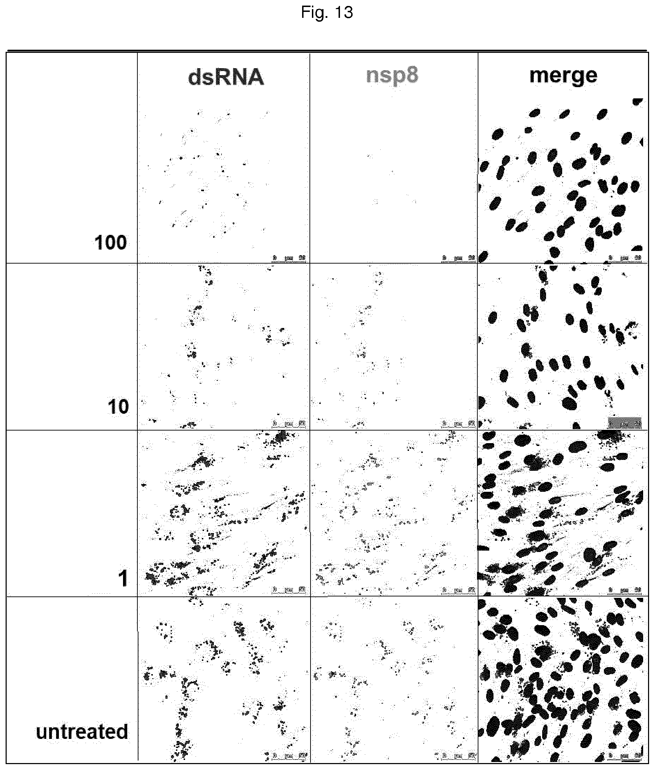

In a series of further experiments, the effects of Silvestrol on HCoV-229E induced formation of replication/transcription complexes (RTCs) and viral protein accumulation were investigated. Therefore, MRC-5 cells were infected with HCoV-229E at a MOI of 3 and treated with the indicated concentrations of Silvestrol or left untreated for 12h p. i.. As shown by immunofluorescence, a dose-dependent antiviral effect of Silvestrol was observed (Fig. 13). The levels of double-stranded viral RNA and the viral protein nsp8 used as markers for RTCs drop down at a concentration of 10 nM Silvestrol. At 100 nM Silvestrol, nearly no viral dsRNA or nsp8 can be detected anymore. This indicates an impaired formation of RTCs in HCoV-229E infected MRC-5 cells treated with concentrations of Silvestrol > 1 nM.

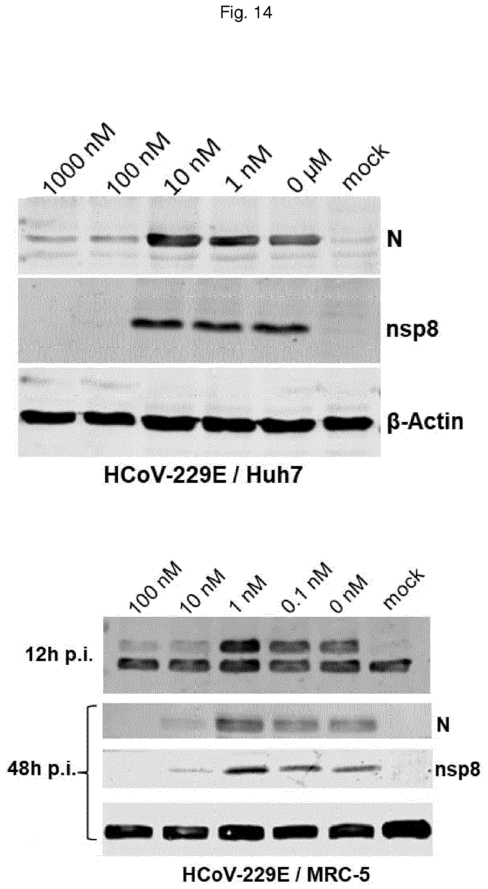

HCoV-229E proteins N and nsp8 are detectable by Westernblotting in Huh-7 infected cells at a Silvestrol concentration of 10 nM. At 100 nM only very low amounts of N-protein can be detected and nsp8 is completely absent at this Silvestrol concentration (Fig. 14). In contrast to that, the level of N-protein already disappears at a concentration of 10 nM Silvestrol in HCoV-229E infected, primary MRC-5 cells (12 h and 48 h p. i.). This substantiates the fact that the EC₅₀ value for Silvestrol is about 10-fold lower in HCoV-229E infected MRC-5 cells (3 nM) compared to infected Huh-7 cells (40 nM) and that a specific antiviral effect of Silvestrol against HCoV-229E can only be seen in primary cells.

The data revealed herein show that the natural compound Silvestrol is a potent antiviral molecule with a broad-spectrum activity. Importantly, Silvestrol shows no major toxic side effects in primary cell systems. This is in line with several studies where Silvestrol was used in different cancer model systems. Thus, Silvestrol-mediated eIF4A inhibition is a novel strategy to combat several pathogenic viruses. For therapeutic intervention, short-term application of low doses of Silvestrol should be an acceptable safety risk to reduce viral titers in the patient. This treatment improves the chances of the immune system to establish an effective antiviral response. It is obvious to the person skilled in the art, that these recent findings, exemplarily performed with Silvestrol, are also valid for Episilvestrol, so that the usage of Episilvestrol is also comprised by the scope of the invention.

Thus it is obvious to the person skilled in the art that the scope of the invention regarding disease control is not confined to the Ebola- and Marburgvirus, but comprises more, if not all, types of RNA viruses which also perform cap-dependent protein translation, e.g. Coronaviridae, Flaviviridae like the Zikavirus and the Denguevirus, Togaviridae like the Chikungunyavirus, Bunyaviridae, Orthomyxo-, Rhabdo-, Paramyxoviridae etc.). This list is, of course, in no way limiting the scope of the invention. The most recent experiments revealed above surprisingly show that Silvestrol and Episilvestrol have also very remarkable antiviral activity against rhinovirus A1 and the poliovirus.

Summarizing the invention, it may be described as follows:
The invention comprises the use of a medicament comprising a therapeutic effective amount of Silvestrol and/or Episilvestrol for the treatment of virus infections of humans and/or mammals.

To the person skilled in the art it is well known that the efficacy of an active ingredient of a medicament may often be improved, i.e. adapted regarding its efficacy, bioavailability, biodistribution, stability and/or compatibility, via chemical modification, e.g. etherification, esterification, sulfonation, substitution, elimination, cyclisation etc. For example, it is well known that fluorination, i.e. substitution of hydrogen atoms by fluorine atoms is prolonging the time span the active ingredient stays within the body of the patient, thus intensifying the efficacy. Therefore it is obvious to the person skilled in the art that any derivative of Silvestrol or Episilvestrol that shows better antiviral effects and/or less unwanted side effects lies within the scope of the invention. A sample list of possible substituents comprises -F, -CI, -Br, -CH3, -C2H5, -C₃H₇, -CF₃, -C2F5, -C₃F₇, -alkyl, -cycloalkyl, -aryl, -heteroaryl, -acyl, -SOₙR_{f}, -SOnH, -POₘR_{f}, -POmH, -SOzN(Rf)2; -POm(NRf)x, -SOz(NRf)y, -B(ORf)2, -OB(ORf)2, -B(ORf)(NRf), -B(NRf)2, -OB(ORf)(NRf), -OB(NRf)2, substituted -aryl, substituted -heteroaryl, -alkylaryl, -alkylheteroaryl, -OH, -phenyl, -NH₂, -NO₂, -OR_{f}, -O-COR_{f}, -CORf, -N(R_{f})₂, -NR_{f}COR_{f}, -O-CSR_{f}, -CSR_{f}, -NR_{f}CSR_{f}, -O-CSeR_{f}, -CSeR_{f}, -NRfCSeR_{f}, -O-CN(R_{f})R_{f}, -CN(Rf)Rf, -NRfCN(Rf)Rf, -N(R_{f})SO₂R_{f}, -N(R_{f})SO₂N(R_{f})₂, -SSRf, -SR_{f}, -SOₓR_{f}, -SOₙN(R_{f})₂, -nitril, -amidine, -guanidine, -COOR_{f}, -CON(R_{f})₂, -CON(Rf)N(Rf)2, -COSR_{f}, -CSOR_{f}, -CSSR_{f}, -NR_{f}COR_{f}, -N(R_{f})N(R_{f})₂, -NHR_{f}, -NR_{f}R_{g}, -CONR_{f}R_{g}, -NHCOR_{f}, -NRfCOR_{g}, -CSRf, -CSNHRf, -CSNRfR_{g}, -CSR_{f}, -OCSR_{f}, -NR_{f}CSR_{f}, -NR_{f}CSR_{g}, -COSR_{f}, -CSNR_{f}N(R_{f})₂, -CONR_{f}N(R_{f})₂, -NR_{f}N(Rf)₂.

Substituents R_{f} and R_{g} are independently from each other chosen from the list comprising -H, -alkyl, -cycloalkyl, -aryl, -heteroaryl, substituted -aryl, substituted -heteroaryl, -alkylaryl, -alkylheteroaryl.

The substituents of the substituted -aryl and substituted -heteroaryl groups are chosen from the list comprising -F, -CI, -Br, -OH, -phenyl, -NH₂, -NO₂, -OR_{f}, -O-COR_{f}, -COR_{f}, -N(R_{f})₂, -NR_{f}COR_{f}, -O-CSR_{f}, -CSR_{f}, -NR_{f}CSR_{f}, -O-CSeR_{f}, -CSeR_{f}, -NR_{f}CSeR_{f}, -O-CN(R_{f})R_{f}, -CN(R_{f})R_{f}, -NR_{f}CN(R_{f})R_{f}, -N(R_{f})SO₂R_{f}, -N(R_{f})SO₂N(R_{f})₂, -SSR_{f}, -SR_{f}, -SOₓR_{f}, -SOₙN(R_{f})₂, -nitrile, -amidine, -guanidine, -COOR_{f}, -CON(R_{f})₂, -CON(R_{f})N(R_{f})₂, -COSR_{f}, -CSOR_{f}, -CSSR_{f}, -NR_{f}COR_{f}, -N(Rf)N(Rf)2 .

The formular indices "x" and "y" can adopt values from 1 to 2, formular index "n" can adopt values from 0 to 4 and formular index "m" can adopt values from 1 to 4.

This list is not limiting the scope of the invention, Silvestrol and Episilvestrol may be multiply substituted, the substituents are chosen independently from each other and may be attached to any part of the base molecule.

Mostly preferred derivatives of Silvestrol and/or Episilvestrol for a medicament according to the invention are derivatives according to the chemical formula I, 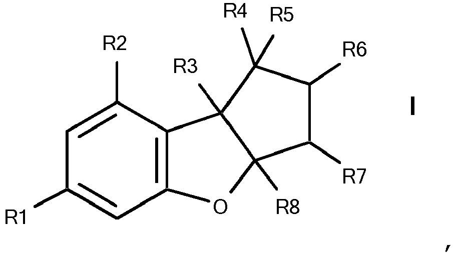 characterized in that R₁ and R₂ are independently chosen from the list comprising F, Cl, Br, Alkyl, Aryl, subst. Alkyl, subst. Aryl, Heteroaryl, subst. Heteroaryl, OH, OR₉, NR₁₀R₁₁, SH, SR₁₂, SOR₁₃, SOR14, COOR₁₅, CONR₁₆R₁₇, SO₂NR₁₈R₁₉, CN, - O-hetero-cycloalkyl, substituted -O-hetero-cycloalkyl and R₃ is independently chosen from the list comprising H, OH, OR₂₀, SR₂₁, NR₂₂R₂₃, F, Cl, Br, Alkyl, and R₄ and R₅ i) are either together representing a substituent chosen from the list comprising =O, =NR24, =NOR25, =CR₂₆R₂₇, =S or ii) R4 is H and R5 is independently chosen from the list comprising OH, NHR24, NHOR25, CHR₂₆R₂₇, SH, SR28 and R₆ is independently chosen from the list comprising the substituents depicted by the chemical formula II through IX, 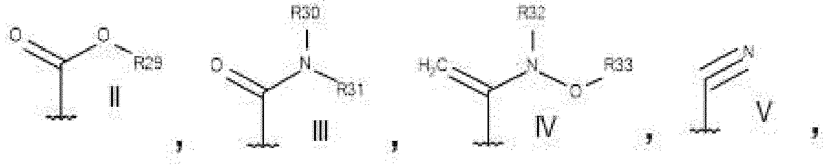 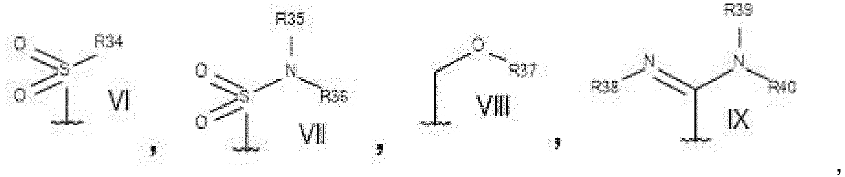 and R₇ and Rs are independently chosen from the list comprising Aryl, subst. Aryl, Heteroaryl, subst. Heteroaryl, whereat R₉ through R₄₀ are independently chosen from the list comprising H, F, Cl, Br, OH, phenyl, NH₂, NO₂, alkyl, branced alkyl, cycloalkyl, aryl, hetero-aryl, substituted alkyl, substituted branched alkyl, substituted cycloalkyl, substituted aryl, substituted heteroaryl, alkylaryl, alkylheteroaryl, nitrile, amidine, guanidine.

Analogously it is well known to the person skilled in the art that so-called analoga of physiologically active substances exert the same biological effect, in this case antiviral activity, although they have chemically different structures. A very common example of this effect is the oestrogenic effect of numerous plasticisers, e.g. diethylphthalate, despite the fact that their chemical structure is very different from the structure of oestrogen. Therefore it is obvious to the person skilled in the art that any analogue of Silvestrol or Episilvestrol that shows similar antiviral effects and/or less unwanted side effects also lies within the scope of the invention. This is especially true if the antiviral effect of the analogue is based on the same mechanism (inhibition of cap-dependent translation).

The invention further comprises the use of a medicament according to the previous paragraph, characterized in that the medicament has an antiviral effect against viruses with cap-dependent or IRES-dependent protein translation and has no specific antiviral effect against viruses with IRES-dependent protein translation, whereat "no specific antiviral effect against viruses with IRES-dependent protein translation" means that efficient inhibition of the virus titer of viruses with IRES-dependent protein translation occurs at concentrations higher than 500 nM, more preferred at concentrations higher than 100 nM and even more preferred at concentrations higher than 50 nM in infected cells. New experiments with the poliovirus and RVA1 revealed an even lower level of effective concentration of Silvestrol/Episilvestrol. Regarding poliovirus an EC₅₀-value of 20 nM is found, regarding RVA1 an EC₅₀-vaule of 100nM. The virus titer considered here is the concentration of the virus within the body fluid relevant for the virus currently under consideration, determined via methods well known to the person skilled in the art. The expression "efficient inhibition" means within this context that the virus titer is reduced for about more than 10 per cent compared to the value without treatment with one or more of the inventive medicaments.

The invention further comprises analogously to the previous paragraph the use of a medicament, characterized in that the efficient inhibition of the virus titer of viruses with IRES-dependent protein translation occurs at EC₅₀-values higher than 10000 nM, more preferred at EC₅₀-values higher than 1000 nM and even more preferred at EC₅₀-values higher than 100 nM in infected cells.

The invention further comprises the use of a medicament according to one or more of the preceding paragraphs, characterized in that the medicament inhibits the propagation of ssRNA viruses with a (+) RNA genome or a (-) RNA genome.

The invention further comprises the use of a medicament according to one or more of the preceding paragraphs, characterized in that it is used in the treatment of a virus infection whereat the virus which is causing the virus infection is a virus with cap-dependent translation.

The invention especially comprises the use of a medicament according to one or more of the preceding paragraphs, characterized in that it is used in the treatment of a virus infection whereat the virus which is causing the virus infection is a virus contained in the list comprising the Ebola virus, the Marburg virus, Coronaviruses, the Zikavirus, the Denguevirus, Togaviridae like the Chikungunyavirus, Bunya-, Orthomyxo-, Rhabdo- and Paramyxoviridae.

The invention further comprises the use of a medicament according to one of the preceding paragraphs, characterized in that it is used in the treatment of a virus infection whereat the virus which is causing the virus infection is a virus with IRES-dependent protein translation.

The invention further comprises the use of a medicament according to the preceding paragraph, characterized in that it is used in the treatment of a virus infection whereat the virus which is causing the virus infection is a virus contained in the list comprising the rhinovirus A1 and the poliovirus.

The invention further comprises the use of a medicament according to one or more of the preceding paragraphs, characterized in that it is applied as a pharmaceutical acceptable composition that can be either inhaled, administered orally, rectally, intravenously, intramuscularly, subcutanously, mucocutaneously, intraperitoneally, intravascularly, transdermally, topically, buccally, intradermally, intra-gastrally, intracutaneously, intranasally, intrabuccally, inthrathecally, percutaneously, sublingually, or can be applied externally as powder, spray, ointment or as medical drops.This list of application techniques is just simply naming some of the most popular application techniques of medicaments exemplarily and thus is not limiting the scope of the invention. It is obvious to the person skilled in the art that other application methods that allow the effective application of the inventive medicament are also included within the scope of the invention. Thus the list of pharmaceutical forms according to the invention comprises drops, oral sprays, nasal sprays, tablets, film-tablets, layer-tablets, suppositories, gels, ointments, syrups, inhalation powders, granulates, emulsions, dispersions, microcapsules, capsules, powder, injection-solutions, infusion-solutions, encapsulations within vesicles as are well known within dermatological/pharmaceutical industry for transporting compounds into and/or through the skin, e.g. anionic or cationic liposomes, niosomes, nano particles, multilamellar vesicles. The multilamellar vesicles may also be used for penetrating cells of skin or hair.

The medicament according to the invention may be produced and/or applied as any of the aforementioned pharmaceutical forms whereat this list is not to be understood as being confining the invention to the mentioned pharmaceutical forms. It is well known to the person skilled in the art that any pharmaceutical form providing sufficient stability to Silvestrol and/or Episilvestrol as well as providing suitable kinetics of releasing of Silvestrol and/or Episilvestrol lies within the scope of the invention.

The invention further comprises the use of a medicament according to one or more of the preceding paragraphs, characterized in that it contains supplementary substances, chosen from the list comprising Citric Acid, Sodium Benzoate, Polyacrylic Acid, Calcium Carbonate, Starch, Lactose Sorbitol, Sucrose, Cellulose, Magnesiumstearate, Dicalciumphosphate, Calciumsulfate, Talc, Mannitol, Ethyl-alcohol, Methylcellulose, Polyvinyl Alcohol, denaturated Gelatine, Sodium-Carboxymethylstarch, natural and/or synthetic Gum like e.g. Arabic Gum, Carob Gum, Karaya Gum, Guar Gum, Tragacanth Gum, Agar, Cellulose derivatives like Methylcellulose, Sodium-Carboxymethylcellulose, microcrystalline Cellulose, Alginate, Alumina, Bentonite, Sugar, Starch from Grain, Rice or Potato, natural Gum like Acacia Gum, Gelatin, Traganth, Alginic Acid, Sodium Alginate, Ammonium-Calcium-Alginate, Methylcellulose, Cera, Sodium-Carboxymethylcellulose, Hydroxypropylmethylcellulose, Polyethylenglycole, Polyvinylpyrrolidone, inorganic compounds like Magnesium-Aluminum-Silicates, Boracic Acid, Stearates e.g. Magnesiumstearat, Calciumstearat, Potassiumstearat, Stearic Acid, high-melting Cera, Sodium-Chloride, Sodium-Benzoate, Sodium-Acetate, Sodium-Oleate, Polyethylenglycol, Amino-Acids like Leucine, Triglycerides of saturated Fatty Acids from plants, e.g. Miglyol 812^{®}, Triglycerin-Diisostearate, Esters of long-chain acids, e.g. Oleyl-Oleate, Isopropylmyristate, Isopropylpalmitate, 2-Ethylhexyl-palmitate, Isooctylstearate, Isopropylstearate, Lauric Acid-Hexylester, Di-n-butyladipate, long-chain alkoholes, e.g. Hexylalcohol, Octylalkohol, Decylalkohol, Oleylalkohol, 2-Octyldodecanol, 2-Hexyldecanol, 2-Octyldecanol.

This list of supplementary substances is just simply naming some of the most popular supplementary substances exemplarily and thus is not limiting the scope of the invention. It is obvious to the person skilled in the art that other supplementary substances that support the storability or applicability of the inventive medicament are also included within the scope of the invention. Additionally the inventive medicament may contain colouring, flavouring, and/or binding agents. Liquid formulations comprise solutions, suspensions, dispersions, sprays and/or emulsions, e.g. aqueous solutions for injections or injection-solutions based on water and Propylenglycole, applicable for parenteral injections.

The aforementioned supplementary substances serve different purposes and are added in varying amounts, e.g.:
- as pharmacologically compatible carrier-substances, amounting from 5 to 95 per cent by weight of the inventive medicament;
- as substance supporting the dissolving of the medicament after application ("blasting means"), amounting from 2 to 30 per cent by weight of the inventive medicament;
- as binding agents, amounting from 1 to 30 per cent by weight of the inventive medicament;
- as lubricant, amounting from 1 to 20 per cent by weight of the inventive medicament;
- as penetrating agent, amounting from 5 to 95 per cent by weight of the inventive medicament

Any supplementary substance may be applied either alone or in combination with at least one other supplementary substance; especially the penetrating agents may be applied either alone or in combination with other penetrating substances. An especially advantageous effect regarding the penetration of medicaments offer long-chain alcohols, mostly 2-alcylic-substituted alcohols. The oils are applied at concentrations ranging from 5 to 95 per cent by weight, more preferred at concentrations ranging from 30 to 90 per cent by weight. The active components (Silvestrol and/or Episilvestrol and/or derivatives of Silvestrol or Episilvestrol and/or analoga of Silvestrol or Episilvestrol) can also be dispersed in water containing emulgated penetration supporting oils, whereat the water-content of these dispersions may be up to 50 per cent by weight, preferredly up to 30 per cent by weight.

The way of dosing of the inventive medicament is determined by the medicating person according to the clinical factors. It is well known to the person skilled in the art that the way of dosing is dependent from numerous criteria, e.g. body size, body weight, body surface, age, gender, the general health situation of the patient, the medicament to be administered, the duration and the way of administering the medicament and also other medicaments which may be administered at the same time, shortly before or afterwards.

The inventive medicament may be combined with at least one other agent, e.g. analgetics and/or antipyretics. Furthermore, it is possible to combine the inventive medicament with at least one anti-inflammatory medicament, immuno-modulator, anti-asthmatic, and/or broncho-dilatator. Also it is possible to combine the inventive medicament with at least one antibacterial, antifungal, anthelmintic and/or antiviral agent. Alternatively or additionally it is possible to combine the inventive medicament with at least one dermatologic and/or cosmetic agent.

The inventive medicament will be - according to the way of its manufacturing/production and its application-technology - used together with liquid and/or solid adjuvants, which are well known to the person skilled in the art, in order to acquire dilutable solutions, emulsions, dissolvable powders, granules, micro-encapsulations in polymeric substances or nano particles. It is - according to the way of administering - manufactured/produced according to a method well known to the person skilled in the art, e.g. by way of thorough mixing and/or grinding of the agents/substances/compounds and/or by addition of solvents and/or solid carrier-substances.

The invention further comprises the use of a medicament according to one or more of the preceding paragraphs, characterized in that it contains the effective amount of Silvestrol and/or Episilvestrol in protonated or deprotonated form, i.e. as salt.

The medicament containing Silvestrol and/or Episilvestrol is advantageously applied after an infection has taken place, but may also be applied in a preventive manner for use in the treatment of humans and/or mammals.

### Description of the drawings

- Fig. 1:: Scheme, showing the formation of the translation initiation complex elF4F that includes the eIF4A helicase activity allowing the bonding (binding) of the 43S preinitiation complex; initiator: Met-charged initiator tRNA.
- Fig. 2A:: Toxicity profile of Silvestrol in the hepatoma cell line Huh-7: Proliferation of Huh-7 cells measured by a WST-1 assay. No effect of Silvestrol on proliferation could be observed up to a concentration of 10 nM. At higher concentrations up to 100 nM Silvestrol, weak effects on proliferation compared to the DMSO control could be observed.
- Fig. 2B:: Antiviral effects of Silvestrol in EBOV-infected Huh-7 cells: Addition of Silvestrol to a final concentration of 10 nM 2 h before viral infection strongly inhibits EBOV replication in Huh-7 cells. Samples from supernatants are collected at days 1, 2, and 3 post infection (p.i.) and subjected to TCID₅₀ analysis in VeroE6 cells. Viral titers determined for EBOV-infected cell cultures in the absence of Silvestrol or DMSO were set to 100%. Statistical significance of the results are indicated by p-values: *: p-value ≤0.05; ***: p-value ≤ 0.001.
- Fig. 2C:: Antiviral effects of Silvestrol in EBOV-infected Huh-7 cells: Morphol-ogy of Huh-7 cells in the light microscope after three days of Silvestrol treatment (10 nM) in the presence or absence of EBOV. Pictures were taken at 10x magnification.
- Fig. 2D:: Antiviral effects of Silvestrol in EBOV-infected Huh-7 cells: Silvestrol inhibits the expression of the EBOV protein VP40, whereas the cellu-lar protein tubulin is not affected. EBOV-infected Huh-7 cells were treated with DMSO or 10 nM Silvestrol 2 h before (lane 2) or immediately post (lane 3) infection. Shown are the levels of the viral protein VP40 and the cellular protein tubulin as control in a Westernblot analysis from samples obtained at 3 days p.i. Mock: uninfected cells without Silvestrol or DMSO addition.
- Fig. 2E:: Downregulation of the Ebola virus proteins GP and NP by Silvestrol in EBOV-infected Huh-7 cells with 10 or 50 nM Silvestrol.
- Fig. 3:: Dose-dependent inhibition of Ebola virus propagation by Silvestrol in Huh-7 cells. Cells are treated with DMSO as a control or with 1, 5 or 10 nM Silvestrol 2 h before EBOV-infection or with 10 nM Silvestrol immediately post EBOV-infection.
- Fig. 4A:: Antiviral effects of Silvestrol in EBOV-infected primary human macrophages: Macrophages from two human donors were infected with EBOV and cell culture supernatants were subjected to TCID₅₀ analysis 3 days post infection. Addition of Silvestrol to 2, 10 or 25 nM 2 h before infection resulted in a concentration-dependent inhibition of EBOV propagation by Silvestrol between 1 to 3 orders of magnitude compared to DMSO-treated cells.
- Fig. 4B:: Antiviral effects of Silvestrol in EBOV-infected primary human macrophages: Similar results (cf. Fig. 4A) are obtained with an enlarged set of EBOV-infected macrophages from seven human donors upon addition of Silvestrol to 10 nM 2 h before infection.
- Fig. 4C:: Antiviral effects of Silvestrol in EBOV-infected primary human macrophages: Westernblot analysis demonstrating strongly decreased levels of the EBOV protein NP and the proto-oncoprotein PIM1 upon treatment of macrophages at 10 nM Silvestrol. Tubulin and β-Actin are shown as cellular control proteins.
- Fig. 4D:: Antiviral effects of Silvestrol in EBOV-infected primary human macrophages: Silvestrol inhibited proliferation of human macrophages with an IC₅₀ value of 96 nM, as determined by a WST-1 viability assay.
- Fig. 5:: Long-term effect of Silvestrol analyzed by Westernblotting. The inhibitory effect of a single-dose treatment with 10 nM Silvestrol on PIM1 levels persists in macrophages up to at least 5 days without affecting β-Actin levels.
- Fig. 6:: Dual Luciferase-assay for analysis of the 5'-UTRs of Ebola mRNAs showing a dose-dependent inhibitory effect of 5 or 10 nM Silvestrol on reporter gene expression. The inhibitory effects of 10 nM Silvestrol on luciferase levels were between 30% for GP and 80% for VP24. Silvestrol has no effect on the IRES-driven expression of the renilla luciferase gene thus allowing normalization of the firefly luciferase levels by renilla luciferase activity (cf. also Fig. 9A/B/C containing more recent experimental data.).
- Fig. 7A:: Effect of Silvestrol in the human lung fibroblast-like cell line MRC5 infected with MERS-CoV (MOI of 0.1). Virus titers are determined 24 hours post transfection. An effective dose (EC₅₀) of 1.3 nM for Silvestrol is determined as well as a CC₅₀-value of 2 µM. (summarized in Fig. 10, Table 1)
- Fig. 7B:: Effect of Silvestrol in the human lung fibroblast-like cell line MRC5 infected with HCoV-229E (MOI of 0.1). Virus titers are determined 24 hours post transfection. An effective dose (EC₅₀) of 3 nM for Silvestrol is determined as well as a CC₅₀-value of 1.7 µM. (summarized in Fig. 10, Table 1)
- Fig. 7C:: Effect of Silvestrol in the human lung fibroblast-like cell line MRC5 infected with MERS-CoV (MOI of 0.1). Virus titers are determined 24 hours post transfection. Bar diagrams showing the inhibitory effect of Silvestrol (virus titers are determined as plaque forming units per ml) in a concentration range between 1 µM and 0.1 nM.
- Fig. 7D:: Effect of Silvestrol in the human lung fibroblast-like cell line MRC5 infected with HCoV-229E (MOI of 0.1). Virus titers are determined 24 hours post transfection. Bar diagrams showing the inhibitory effect of Silvestrol (virus titers are determined as plaque forming units per ml) in a concentration range between 1 µM and 0,1 nM.
- Fig. 7E:: Determination of cell cytotoxicity of Silvestrol in MRC5 cells. CC₅₀ values are about 2 µM for Silvestrol at 37°C (conditions for MERS-CoV infection). (summarized in Fig. 10, Table 1)
- Fig. 7F:: Determination of cell cytotoxicity of Silvestrol in MRC5 cells. CC₅₀ values are about 1.7 µM at 33°C (conditions for HCoV-229E infection). (summarized in Fig. 10, Table 1)
- Fig. 7G:: Effect of Silvestrol upon IRES-dependent translation on cell line MRC5, infected with Rhinovirus 1A (24h post infection), showing an EC₅₀-value of 0.1 µM and a CC₅₀-value of 1.7 µM. (summarized in Fig. 10, Table 1)
- Fig. 8A:: Effect of Silvestrol in HeLa cells infected with Rhinovirus 1A (MOI of 0.1). Virus titers are determined 24 hours post transfection. An effective dose (EC₅₀) of 0.4 µM for Silvestrol is determined.
- Fig. 8B:: Effect of Silvestrol in HeLa cells infected with Rhinovirus 1A (MOI of 0.1). Virus titers are determined 24 hours post transfection. Bar diagrams showing the inhibitory effect of Silvestrol (virus titers are determined as plaque forming units per ml) in a concentration range between 10 µM and 0.01 nM.
- Fig. 8C:: Effect of Silvestrol in Huh-7 cells infected with HCoV-229E (MOI of 0.1). Virus titers are determined 24 hours post transfection. An effective dose (EC₅₀) of 0.04 µM for Silvestrol is determined.
- Fig. 8D:: Effect of Silvestrol in Huh-7 cells infected with HCoV-229E (MOI of 0.1). Virus titers are determined 24 hours post transfection. Bar diagrams showing the inhibitory effect of Silvestrol (virus titers are determined as plaque forming units per ml) in a concentration range between 10 µM and 0.01 nM.
- Fig. 8E:: Effect of Silvestrol in VeroE6 cells infected with Poliovirus (MOI of 0.1). Virus titers are determined 24 hours post transfection. An effective dose (EC₅₀) of 0.1 µM for Silvestrol is determined.
- Fig. 8F:: Effect of Silvestrol in VeroE6 cells infected with Poliovirus (MOI of 0.1). Virus titers are determined 24 hours post transfection. Bar diagrams showing the inhibitory effect of Silvestrol (virus titers are determined as plaque forming units per ml) in a concentration range between 1 µM and 0.01 nM.
- Fig. 9A:: Analysis of Silvestrol effects on reporter gene activity mediated by viral 5'-UTRs: Schematic presentation of the dual luciferase reporter vector. The HCV IRES-driven expression of the Renilla luciferase is eIF4A-independent and is used to normalize transfection efficiencies.
- Fig. 9B:: Analysis of Silvestrol effects on reporter gene activity mediated by viral 5'-UTRs: Effects of 5 and 10 nM Silvestrol on Firefly luciferase activity. The seven 5'-UTRs of EBOV are cloned upstream of the reporter gene. The 5'-UTRs of the β-globin and the β-actin mRNAs are used as negative controls. The 5'-UTR of PIM1 serves as a positive control. All measured values are normalized to DMSO. Standard errors of the mean of at least 8 independent experiments are shown.
- Fig. 9C:: Analysis of Silvestrol effects on reporter gene activity mediated by viral 5'-UTRs: Effects of Silvestrol on reporter gene expression in the context of 5'-UTRs from coronaviruses HCoV-229E and MERS-CoV. All measured values are normalized to DMSO. Standard errors of the mean of at least 8 independent experiments are shown.
- Fig. 10:: Table 1: CC₅₀ and EC₅₀ values determined for silvestrol-treated cells that were mock infected (CC₅₀) or infected with the indicated viruses (EC₅₀). MTT assays were performed at 37 ºC except for cells used for HCoV-229E and HRV A1 infections, which were performed at 33 ºC (indicated by asterisks). SI, selectivity index: CC₅₀ divided by EC₅₀.
- Fig. 11:: Antiviral activity of Silvestrol against (+)RNA-strand viruses with Capdependent and IRES-driven viral translation. (**A**) Primary MRC-5 cells are infected with the coronaviruses MERS-CoV or HCoV-229E (MOI = 0.1) and incubated with different Silvestrol concentrations. (**B**) MRC-5 cells are infected with the picornaviruses rhinovirus 1A or poliovirus and incubated with the indicated Silvestrol concentrations. (**C**) Primary hAEC and PBMCs are infected with MERS-CoV or HCoV-229E as indicated. After 24 h the viral titers (given in percentage) are determined to calculate EC₅₀ values (n=3).
- Fig. 12: Antiviral activity of Silvestrol in HCoV-229E infected PBMCs. (**A**) Effects of different Silvestrol concentrations on virus titers and (**B**) calculation of the EC₅₀ value from two donors. PBMCs were infected with an MOI of 0.1. (**C**) Determination of the CC₅₀ value in PBMCs. A selectivity index of 133 was determined for Donor 1.
- Fig. 13:: Immunofluorescence to analyze the effects of Silvestrol on viral dsRNA and nsp8 levels in HCoV-229E-infected MRC-5 cells. Cells are infected with a MOI of 3 and incubated with the indicated Silvestrol concentrations. The cells are fixed 12 h p. i. using methanol and analyzed using confocal microscopy using specific antibodies for dsRNA (left column) and nonstructural protein 8 (nsp 8, center column). For visualization of the formation of viral RTCs Alexa Fluor 594 goat anti-mouse IgGand Alexa Fluor 488 F(ab')2 fragment of goat anti-rabbit IgG is used (n = 2). (Experimental results shown with background color inverted (black → white) for purpose of reproduction).
- Fig. 14:: Western blot experiments to analyse the effects of Silvestrol on protein levels of HCoV-229E nucleocapsid protein (N) and nonstructural protein 8 (nsp8). Huh7 and MRC-5 cells are infected with HCoV-229E (MOI = 3) and treated with the indicated concentrations of Silvestrol for 12 h p. i. Cell lysates and western blots are done as described. As loading control served β-Actin (n=3).
- Fig. 15A:: Basic mechanism of viral translation initiation; example Coronavirus (Cap-dependent translation initiation)
- Fig. 15B:: Mechanism of cap-dependent translation initiation via association of the elF2 ternary complex with the 40S subunit complex to form a 43S pre-initiation complex.
- Fig. 15C:: Mechanism of cap-dependent translation initiation via mRNA-activation.
- Fig. 15D:: Mechanism of 5'-cap-dependent translation-initiation via binding of the 43S pre-initiation-complex to mRNA, followed by 5' → 3' scanning (involving eIF4A helicase activity) and subsequent recognition of the start codon.
- Fig. 16:: Overview of the type-specific structure elements and initiation factors involved in the different mechanisms of IRES-dependent translation initiation, as is well known in the state of the art:
i) Type I IRES (recruitment of the 43S complex to the Internal Ribosome Entry Site and scanning of the 5'-UTR until the AUG start codon is reached).
ii) Type II IRES (recruitment of the 43S complex into the direct vicinity of the AUG start codon, no scanning necessary).
iii) Type III IRES (recruitment of the 43S complex directly onto the AUG start codon).
iv) Type IV IRES (without any need for canonical initiation factors).

### Detailed embodiments of the invention

### Cells and viruses

Human hepatocyte derived cellular carcinoma cells (Huh7), African Green monkey kidney cells (VeroE6, "Vero cells"), human cervical cancer cells (Hela) and primary lung fibroblasts (MRC-5) are grown in Dulbecco modified Eagle medium (DMEM) supplemented with 10 % FCS (fetal calf serum), 100 U/ml penicillin and 100 µg/ml streptomycin at 37° C and 5 % CO₂.

HepG2 cells are cultured in Iscove's Modified Dulbecco's Medium (IMDM) supplemented with 10 % fetal calf serum (FCS) at 37°C and 5 % CO₂. Human Coronavirus 229E (HCoV-229E), MERS-CoV (EMC/2012), human Rhinovirus 1A (HRV1A), rhinovirus 1A (RV1A) and Poliovirus Typ 1 (strain Mahoney; PV) are provided by the virus strain collection of the Institute of Medical Virology, Giessen, Germany.

### Reagent

Silvestrol, a natural inhibitor was obtained from Medchemexpress (LLC, Princton, US; purity >98 %). Stock solution of 6 mM was prepared in DMSO and further diluted in DMEM respectively IMDM. Control cells were treated with corresponding DMSO dilutions lacking silvestrol.

### Ebola virus infection

All work with Zaire Ebola virus (EBOV) strain Mayinga (Acession number AF 086833) is performed at the biosafety level 4 (BSL4) laboratory of the Philipps University Marburg. Huh-7 cells (5 × 10⁵ cells) or primary human macrophages (3 × 10⁶ cells) are infected with EBOV at a multiplicity of infection (MOI) of 0.1 for 1 h at 37°C. Then Inoculum is removed and cells are incubated in Dulbecco's modified Eagle medium (DMEM) containing 3% fetal calf serum supplemented with Silvestrol or DMSO as indicated.

Aliquots of the supernatant are collected at 1-4 days post infection (p.i.) and subjected to the 50% tissue culture infectious dose TCID₅₀ assay for determination of viral titers. Cells are lysed with 1% SDS, followed by Westernblotting to analyze for expression of viral proteins and the cellular PIM1 kinase.

### Silvestrol treatment of EBOV-infected cells

Silvestrol is added to cell cultures as a single dose to final concentrations of 1 to 25 nM (dissolved from a 6 mM stock in DMSO, dilutions in DMEM). Silvestrol is added (Huh-7: one day post seeding; primary human macrophages: 7 days post seeding) 2 h before infection or directly post infection. As a control, DMSO is added to cells at the same concentration used to dissolve Silvestrol. Infected cells are incubated for 1 to 4 days at 37°C in growth medium containing the indicated Silvestrol concentrations.

### TCID₅₀ analysis

Virus titers in the supernatant of infected cells are determined by the 50% tissue culture infectious dose (TCID₅₀) assay in VeroE6 cells as is well known to the person skilled in the art.

### Cell culture and preparation of macrophages from PBMCs

Huh-7 (human hepatoma) and VeroE6 (African green monkey, kidney epithelia) cells are cultivated in Dulbecco's modified Eagle medium complemented with penicillin (100 U/ml), streptomycin (100 mg/ml), 5 mM L-glutamine and 10% fetal calf serum at 37°C and 5% CO₂ in a humidified atmosphere.

Human PBMCs (Peripheral Blood Mononuclear Cells) are isolated from buffy coats by density gradient centrifugation and magnetic CD14 MicroBeads according to the manufacturer's protocol (Miltenyi Biotec, Bergisch Gladbach, Germany). Monocytes (3 × 10⁶ cells) are cultivated in 6-well tissue culture plates (Falcon Primaria, Becton Dickinson, Paramus, NJ) at 37°C in 5% CO₂ using RPMI 1640 medium (GE Healthcare, Freiburg, Germany) supplemented with 2 mM L-Glutamine, 1 mM sodium pyruvate, 100 U/ml penicillin, 100 µg/ml streptomycin and 1% non-essential amino acids. After 2 h, defibrinated human AB serum is added to a final concentration of 2%, and GM-CSF (Biochrom, Berlin, Germany) to a final concentration of 10 ng/ml. Cells are cultivated for 7 days to differentiate them into macrophages. On day 3 post seeding half of the medium is replaced with fresh medium (including all supplements). Alternatively, preparation of monocytes from PBMCs is performed by counterflow centrifugation.

### WST-1 assay of human Huh-7 cells or primary macrophages

Huh-7 cells (7 × 10³ cells in 200 µl IMDM, 10% FCS) are seeded in 96-well microplates and incubated for 24 h (37°C, 5% CO₂). The medium is replaced with fresh medium, and Silvestrol or DMSO is added as indicated for 48 h. The medium is aspirated and 110 µL of 10% WST-1 reagent (Roche, Mannheim, Germany) in PBS is added. Absorbance is measured using a Tecan Safire II (measurement wavelength: 450 nm; reference wavelength: 600 nm).

Monocytes (8 × 10⁴ cells in 100 µL RPMI 1640) are seeded in 96-well microplates and incubated for 2 h (37°C, 5% CO₂). Then 100 µL RPMI 1640 supplemented with defibrinated human AB serum (final concentration 2%) and GM-CSF (final concentration 10 ng/mL) (Biochrom, Berlin, Germany) is added. After 72 h, half of the medium is removed and replaced with RPMI 1640 containing 4% human serum. After 96 h, the monocytes are differentiated into macrophages. The medium is then replaced with RPMI 1640 containing 2% human serum, and Silvestrol or DMSO is added as indicated followed by incubation for another 72 h. The WST-1 assay is then performed as described above for Huh-7 cells.

### Westernblot analysis, Method 1

Cells are lysed in lysis buffer (125 mM Tris/HCI pH 6.8, 4% SDS, 1.4 M 2-mercaptoethanol, 0.05% bromophenol blue) and heated at 95 °C for 5 min. Samples are loaded onto 15% SDS-polyacrylamide gels followed by electrophoresis for 1 h at 180 V. Proteins are transferred onto an Immobilon-P PVDF membrane (Merck Millipore, Darmstadt, Germany) for 30 min at 10 V followed by blocking of the membrane with 5% milk powder dissolved in TBST (10 mM Tris/HCI, 150 mM NaCl, 0,1% Tween 20, pH 7.6). Primary and secondary antibodies are diluted 1:500 in TBST (Pim1, sc-13513, Santa Cruz Biotechnology), 1:5000 (β-Actin, sc-47778, Santa Cruz Biotechnology) and 1:5000 (goat anti-mouse IgG-HRP, secondary antibody). Blots are incubated with Amersham ECL^{™} or ECLplus^{™} Western Blotting Detection Reagents according to the manufacturer's protocol. For detection of chemiluminescence, Kodak^{®} BioMaxTM light films, Kodak GBX Developer and Replenisher and GBX Fixer and Replenisher are used.

Samples from infected cells are lysed in 1% SDS containing sample buffer and subjected to 12% SDS PAGE and semi-dry transfer onto nitrocellulose membranes. Staining of viral proteins from infected Huh-7 cells is performed using a goat anti-VP40 antibody and a donkey anti-goat Alexa680nm antibody (Thermo Fisher Scientific/Molecular Probes). Detection of antibodies is performed using the Odyssey Infrared Imaging System (LI-COR, Lincoln, NE, USA). Staining of viral proteins from human primary macrophages is performed using a chicken anti-NP antibody (at a 1:1000 dilution) and a donkey anti-chicken peroxidase-coupled antibody (Dianova). Tubulin is stained using mouse anti-tubulin (SIGMA) and goat anti-mouse peroxidase-coupled antibody (DAKO). Detection of antibodies is performed using the ChemiDoc^{™} XRS+ System (BIO-RAD).

### Westernblot analysis, Method 2

Viral protein accumulation was analyzed by Western blot analysis. MRC-5 or Huh-7 cells were infected with HCoV-229E at a MOI of 3. At 1 h p.i., the indicated concentrations of silvestrol in DMEM supplemented with antibiotics were added to the cells for 12 h and 48 h, respectively. The cells were lysed with NP40-containing buffer (150 mM NaCl, 50 mM Tris-HCI, pH 7.5, 1 % NP40, 1x protease inhibitor cocktail [Sigma-Aldrich]). Proteins were separated by 10 % SDS-PAGE and blotted onto a nitrocellulose membrane (Amersham). Membranes were incubated with mouse anti-nucleocapsid protein mAb (Ingenasa), rabbit antiserum specific for HCoV-229E nonstructural protein 8 (nsp8) and rabbit anti-actin antibody (abeam), respectively, each diluted 1:1000 in PBS containing 1 % bovine serum albumin (BSA). Following incubation for 60 min, the membranes were extensively washed with PBS and incubated with appropriate secondary antibodies (IRDye-conjugated anti-mouse and anti-rabbit mAb [Li-Cor], respectively) diluted 1:10.000 in PBS containing 1 % BSA. After 1 h, membranes were washed and analyzed using the LI-COR Odyssey imaging system.

### Antiviral activity of Silvestrol against Coronaviruses

MRC-5 cells are infected with the low pathogenic HCoV-229E or with the high pathogenic MERS-CoV at a MOI of 0.1. The cell cytotoxicity of Silvestrol in MRC-5 cells is analyzed at 33°C (temperature optimum for HCoV-229E) and 37°C (temperature optimum for MERS-CoV) and the cell cytotoxicity is measured by determining CC₅₀ values of about more than 1 µM under both conditions. Importantely, low doses of Silvestrol efficiently reduce the viral titers resulting in an EC₅₀ value of 1.3 nM for MERS-CoV infected- and an EC₅₀ value 3 nM for HCoV-229E infected MRC-5 cells. Thus, a selectivity index (CC₅₀/EC₅₀) for Silvestrol of about 500 is observed.

### Dual-Luciferase Reporter Assay, Method 1 (cf. Fig. 6)

The day before transfection 2×10⁴ HepG2 cells per well are seeded in a 98-well plate (Greiner Bio One International GmbH, cat. #655090) in 200 µL IMDM (Lonza, cat. #12-722F) supplemented with 10% FCS (Biochrom AG, cat. #S0115) and incubated at 37°C, 5% CO₂. The transfection is done in 100 µL Opti-MEM (Thermo Fisher Scientific Inc., cat. #51985026). 0.5 µg of the reporter construct DNA is diluted in 50 µL of Opti-MEM. 0.5 µL of Lipofectamine^{®} 2000 Transfection Reagent (Thermo Fisher Scientific Inc., cat. #11668019) is used per transfection and therefore diluted in 50 µL of Opti-MEM. After 5min of incubation at room temperature both components are mixed and incubated for another 15 min at room temperature. Then complexes are added to the Opti-MEM media in the wells and incubated at 37°C, 5% CO₂. 4-6 h later the medium is aspirated and substituted with fresh medium (IMDM + 10 % FCS) containing the determined concentration of Silvestrol in sterile filtrated DMSO (Carl Roth GmbH + Co. KG, cat. #4720.4) respectively DMSO only. The cells are incubated for 48 h at 37°C, 5% CO₂ before performing the Dual-Luciferase^{®} Reporter Assay System (Promega Corporation, cat. #1960) according to the manual instructions. Measurements are done in the Tecan Safire 2 Multimode Reader.

### Dual-Luciferase Reporter Assay, Method 2

The day before transfection, 2×10⁴ HepG2 cells per well were seeded in a 96-well plate (Greiner Bio One International GmbH) in 200 µL IMDM (Lonza) supplemented with 10 % FCS (Biochrom AG) and incubated at 37°C, 5 % CO₂. The transfection was performed using 100 µL Opti-MEM (Thermo Fisher Scientific Inc.). For transfection of five wells, 0.5 µg of the reporter construct DNA were diluted in 25 µL of Opti-MEM. In parallel, 0.5 µL of Lipofectamine^{®} 2000 Transfection Reagent (Thermo Fisher Scientific Inc.) were mixed with 24.5 µL of Opti-MEM. Both samples were preincubated for 5 min at room temperature, then combined and incubated for another 15 min at room temperature. Then, 10 µL of the solution were added to each well containing the cells in 100 µL Opti-MEM medium. The plates were incubated at 37°C and 5 % CO₂. 4-6 h posttransfection (p.t.), the medium was aspirated and substituted with 200 µL fresh medium (IMDM + 10 % FCS) containing the appropriate silvestrol concentration or an equivalent amount of DMSO but lacking silvestrol. Following incubation of the cells for 48 h at 37°C at 5 % CO₂, a Dual-Luciferase^{®} Reporter Assay (Promega Corporation) was performed according to the manufacturer's instructions. Measurements were done using a Tecan Safire 2 Multimode Reader.

### Antiviral Assays / Antiviral activity, Method 1

To analyse the antiviral activity of Silvestrol, confluent monolayers (48 well format, 1,9×10⁴ cells per well) of the respective cell lines are infected with HCoV-229E, MERS-CoV (on Huh7- and MRC-5 cells), HRV1A (on Hela- and MRC-5 cells) or PV (on Vero- and MRC-5 cells) with an MOI of 0.1. For HCoV-229E and HRV1A the cells are incubated at 33°C, whereas MERS-CoV and PV are incubated at 37°C. After 1 h of absorption the virus inoculum is removed, cells are rinsed with PBS and fresh medium without FCS containing the indicated concentrations of Silvestrol or corresponding DMSO solvent is added. 24 h p.i. supernatant was collected and stored at -80°C till assayed.

### Antiviral Assays / Antiviral activity, Method 2:

To determine the effective concentration 50 (EC₅₀) of Silvestrol at which 50 % of the virus titer is reduced, corresponding cell lines are inoculated with an MOI of 0.1 for 1 h in PBS/BA/P/S (PBS containing 0.2 % BSA, 1 mM MgCl₂, 0.9 mM CaCl₂, 100 U/ml penicillin and 100 mg/ml streptomycin) at 33°C (for HCoV-229E, RV1A) or 37°C (for MERS-CoV and PV). After removing the inoculums, cells are incubated with DMEM containing antibiotics and different inhibitor concentrations. Supernatants are collected after 24 h post infection (p.i.) and virus titers are further analyzed using plaque assay, Method 2.

### Plaque Assay, Method 1:

Virus titers are determined via plaque assay. Therefore Hela cells (for RV), Vero cells (for PV), Huh7 cells (for HCoV-229E and MERS-CoV) are seeded in 6-well plates (cells should be 90-100% confluent, ∼9,5×10⁵) and inoculated with 10-fold virus dilutions in PBS++/BA/P/S (PBS containing 0.2 % BSA, 1 mM MgCl₂, 0.9 mM CaCl₂, 100 U/ml penicillin and 100 mg/ml streptomycin) for 1 h. Afterwards Avicel-containing medium (1 × MEM, 1.25 % Avicel, FMC Biopolymer) is added to the cells. After 2-4 d incubation (depending on virus type) the plates are washed with PBS, fixed with 3.7 % PFA in PBS and the cell layer is stained with 0.15 % Crystal violet in PBS. Effective concentration 50 (EC50) is calculated using GraphPad Prism 5.0.

### Plaque Assay, Method 2:

Corresponding cells are seeded in 24 well plates and inoculated with 10-fold virus dilution in PBS/BA/P/S for 1h. Afterwards Avicel-containing medium (1×MEM [Gibco], 1.25 % Avicel [FMC Biopolymer]) is added. After 2-4 d incubation the plates are washed with PBS, fixed with 3.7 % PFA in PBS and the cell layer is stained with 0.15 % Crystal violet.

In order to define the EC₅₀ the viral titer of the untreated, virus-infected control is set at 100 % and the titers of silvestrol-treated samples are calculated in relation to it.

### Cell toxicity

The toxicity of Silvestrol is evaluated by incubation of the respective cells (cells should be 90-100% confluent, ∼1×10⁴ cells per well) with serial dilution of the compound (Silvestrol) in DMEM supplemented with 100 U/ml penicillin and 100 µg/ml streptomycin for 24 or 48 h in 96 well formats. Afterwards the medium is exchanged by 200 µl of MTT-mix (DMEM supplemented with 10 % FCS containing 175 µg/ml or 250 µg/ml tetrazolium bromide, Sigma), which is added to each well. Cells are then further incubated for 90-120 Min. at 37°C and subsequently fixed in 3.7 % PFA (in PBS) for 30 min. Fixing solution is aspirated and the cells are air dried. Tetrazolium crystals are dissolved by adding 200 µl isopropanol to each well and photometrically analyzed at 490 nm excitation by ELISA reader (BioTek). Cytotoxic concentration 50 (CC₅₀) is calculated using GraphPad Prism 5.0.

### Immunofluorescence

Immunofluorescence was performed as is known in the state of the art. Briefly, MRC-5 cells were infected with HCoV-229E (MOI = 3) and treated with indicated concentrations of silvestrol or left untreated for 12 h p.i.. Afterwards cells are fixed with ice-cold methanol and stained with mouse anti-dsRNA mAb (J2, English& Scientific Consulting bt) and polyclonal rabbit anti-HCoV-229E non-structural protein 8 (nsp8) serum. For detection Alexa Fluor 594 goat anti-mouse IgG and Alexa Fluor 488 F(ab')2 fragment of goat anti-rabbit IgG (Invitrogen, USA) were used. Confocal microscopy was done using Leica SP05 CLSM and LAS-AF software (Leica).

## Claims

1. A medicament comprising a therapeutic effective amount of Silvestrol and/or Episilvestrol for use in the treatment of virus infections of humans and/or mammals with ssRNA viruses with a (+) RNA genome or a (-) RNA genome whereat the virus which is causing the virus infection is a virus contained in the list comprising the Ebola virus, the Marburg virus, Coronaviruses, Filoviridae.

2. A medicament for use in the treatment of virus infections of humans and/or mammals according to the preceding claim, **characterized in that** it is applied as a pharmaceutical acceptable composition that can be either inhaled, administered orally, rectally, intravenously, intramuscularly, subcutanously, intraperitoneally, intravascularly, or can be applied externally as powder, spray, ointment or as medical drops.

3. A medicament for use in the treatment of virus infections of humans and/or mammals according to one or more of the preceding claims, **characterized in that** it contains supplementary substances, chosen from the list comprising Citric Acid, Sodium Benzoate, Polyacrylic Acid, Calcium Carbonate, Starch, Lactose Sorbitol, Sucrose, Cellulose, Magnesiumstearate, Dicalciumphosphate, Calciumsulfate, Talc, Mannitol, Ethyl-alcohol, Methylcellulose, Polyvinyl Alcohol, denaturated Gelatine, Sodium-Carboxymethylstarch, natural and/or synthetic Gum like e.g. Arabic Gum, Carob Gum, Karaya Gum, Guar Gum, Tragacanth Gum, Agar, Cellulose derivatives like Methylcellulose, Sodium-Carboxymethylcellulose, microcrystalline Cellulose, Alginate, Alumina, Bentonite, Sugar, Starch from Grain, Rice or Potato, natural Gum like Acacia Gum, Gelatin, Traganth, Alginic Acid, Sodium Alginate, Ammonium-Calcium-Alginate, Methylcellulose, Cera, Sodium-Carboxymethylcellulose, Hydroxypropylmethylcellulose, Polyethylenglycole, Polyvinylpyrrolidone, inorganic compounds like Magnesium-Aluminum-Silicates, Boracic Acid, Stearates e.g. Magnesiumstearat, Calciumstearat, Potassiumstearat, Stearic Acid, high-melting Cera, Sodium-Chloride, Sodium-Benzoate, Sodium-Acetate, Sodium-Oleate, Polyethylenglycol, Amino-Acids like Leucine, Triglycerides of saturated Fatty Acids from plants, e.g. Triglycerin-Diisostearate, Esters of long-chain acids, e.g. Oleyl-Oleate, Isopropylmyristate, Isopropylpalmitate, 2-Ethylhexyl-palmitate, Isooctylstearate, Isopropylstearate, Lauric Acid-Hexylester, Di-n-butyladipate, long-chain alkoholes, e.g. Hexylalcohol, Octylalkohol, Decylalkohol, Oleylalkohol, 2-Octyldodecanol, 2-Hexyldecanol, 2-Octyldecanol.

4. A medicament for use in the treatment of virus infections of humans and/or mammals according to one or more of the preceding claims, **characterized in that** it contains the effective amount of Silvestrol and/or Episilvestrol in protonated or deprotonated form, i.e. as salt.

5. A medicament for use in the treatment of virus infections of humans and/or mammals according to one or more of the preceding claims, **characterized in that** it is applied in a preventive manner for use in the treatment of humans and/or mammals.

## Patentansprüche

1. Ein Medikament, umfassend eine therapeutisch wirksame Menge von Silvestrol und/oder Episilvestrol zur Verwendung bei der Behandlung von Virusinfektionen von Menschen und/oder Säugetieren mit ssRNA-Viren mit einem (+) RNA-Genom oder einem (-) RNA-Genom, wobei das Virus, das die Virusinfektion verursacht, ein Virus ist, das in der Liste enthalten ist, die das Ebola-Virus, das Marburg-Virus, Coronaviren, Filoviridae umfasst.

2. Ein Medikament zur Verwendung bei der Behandlung von Virusinfektionen bei Menschen und/oder Säugetieren nach dem vorhergehenden Anspruch, **dadurch gekennzeichnet, dass** es als pharmazeutisch akzeptable Zusammensetzung angewendet wird, die entweder inhaliert oder oral, rektal, intravenös, intramuskulär, subkutan, intraperitoneal, intravaskulär verabreicht werden kann, oder äußerlich als Pulver, Spray, Salbe oder als medizinische Tropfen angewendet werden kann.

3. Ein Medikament zur Verwendung bei der Behandlung von Virusinfektionen von Menschen und/oder Säugetieren nach einem oder mehreren der vorhergehenden Ansprüche, **dadurch gekennzeichnet, dass** es ergänzende Substanzen enthält, ausgewählt aus der Liste umfassend Zitronensäure, Natriumbenzoat, Polyacrylsäure, Calciumcarbonat, Stärke, Lactose, Sorbit, Saccharose, Cellulose, Magnesiumstearat, Dicalciumphosphat, Calciumsulfat, Talkum, Mannitol, Ethylalkohol, Methylcellulose, Polyvinylalkohol, denaturierte Gelatine, NatriumCarboxymethylstärke, natürliches und/oder synthetisches Gummi wie z. B. Gummi Arabicum, Johannisbrotkernmehl, Karayagummi, Guarkernmehl, Tragantgummi, Agar, Cellulosederivate wie Methylcellulose, Natrium-Carboxymethylcellulose, mikrokristalline Cellulose, Alginat, Tonerde, Bentonit, Zucker, Stärke aus Getreide, Reis oder Kartoffeln, natürliches Gummi wie Akaziengummi, Gelatine, Traganth, Alginsäure, Natriumalginat, Ammonium-Calcium-Alginat, Methylcellulose, Cera, Natrium-Carboxymethylcellulose, Hydroxypropylmethylcellulose, Polyethylenglycole, Polyvinylpyrrolidon, anorganische Verbindungen wie Magnesium-Aluminium-Silikate, Borsäure, Stearate, z. B. Magnesiumstearat, Calciumstearat, Kaliumstearat, Stearinsäure, hochschmelzende Cera, Natriumchlorid, Natriumbenzoat, Natriumacetat, Natriumoleat, Polyethylenglykol, Aminosäuren wie Leucin, Triglyceride gesättigter Fettsäuren aus Pflanzen, z. B. Triglycerin-Diisostearat, Ester langkettiger Säuren, z. z. B. Oleyloleat, Isopropylmyristat, Isopropylpalmitat, 2-Ethylhexylpalmitat, Isooctylstearat, Isopropylstearat, Laurinsäure-Hexylester, Di-n-butyladipat, langkettige Alkohole, z. B. Hexylalkohol, Octylalkohol, Decylalkohol, Oleylalkohol, 2-Octyldodecanol, 2-Hexyldecanol, 2-Octyldecanol.

4. Ein Medikament zur Verwendung bei der Behandlung von Virusinfektionen von Menschen und/oder Säugetieren nach einem oder mehreren der vorhergehenden Ansprüche, **dadurch gekennzeichnet, daß** es die wirksame Menge an Silvestrol und/oder Episilvestrol in protonierter oder deprotonierter Form, d.h. als Salz, enthält.

5. Ein Medikament zur Verwendung bei der Behandlung von Virusinfektionen von Menschen und/oder Säugetieren nach einem oder mehreren der vorhergehenden Ansprüche, **dadurch gekennzeichnet, dass** es zur Verwendung bei der Behandlung von Menschen und/oder Säugetieren präventiv angewendet wird.

## Revendications

1. Médicament comprenant une quantité thérapeutiquement efficace de Silvestrol et/ou d'Episilvestrol pour une utilisation dans le traitement d'infections virales d'humains et/ou de mammifères avec des virus ssRNA avec un génome ARN (+) ou un génome ARN (-) où le virus qui cause l'infection virale est un virus contenu dans la liste comprenant le virus Ebola, le virus Marburg, les Coronavirus, les Filoviridae.

2. Médicament destiné à être utilisé dans le traitement des infections virales de l'homme et/ou des mammifères selon la revendication précédente, **caractérisé en ce qu'**il est appliqué sous la forme d'une composition pharmaceutiquement acceptable qui peut être soit inhalée, administrée par voie orale, rectale, intraveineuse, intramusculaire, sous-cutanée, intrapéritonéale, intravasculaire, soit appliquée par voie externe sous forme de poudre, de spray, de pommade ou de gouttes médicales.

3. Médicament pour utilisation dans le traitement des infections virales de l'homme et/ou des mammifères selon une ou plusieurs des revendications précédentes, **caractérisé en ce qu'**il contient des substances supplémentaires, choisies dans la liste comprenant l'acide citrique, le benzoate de sodium, l'acide polyacrylique, le carbonate de calcium, l'amidon, le lactose, le sorbitol, le saccharose, la cellulose, le stéarate de magnésium, le dicalciumphosphate, le calciumsulfate, le talc, le mannitol, l'alcool éthylique, la méthylcellulose, l'alcool polyvinylique, la gélatine dénaturée, le carboxyméthylamidon sodique, les gommes naturelles et/ou synthétiques, par ex. Gomme arabique, gomme de caroube, gomme de karaya, gomme de guar, gomme adragante, agar, dérivés de la cellulose comme la méthylcellulose, la carboxyméthylcellulose de sodium, la cellulose microcristalline, l'alginate, l'alumine, la bentonite, le sucre, l'amidon de céréales, de riz ou de pomme de terre, la gomme naturelle comme la gomme d'acacia, la gélatine, Traganth, acide alginique, alginate de sodium, alginate d'ammonium et de calcium, méthylcellulose, céra, carboxyméthylcellulose de sodium, hydroxypropylméthylcellulose, polyéthylèneglycole, polyvinylpyrrolidone, composés inorganiques comme les silicates d'aluminium et de magnésium, acide boracique, stéarates, par exemple stéarate de magnésium, stéarate de calcium, stéarate de potassium, acide stéarique, cères à haut point de fusion, chlorure de sodium, benzoate de sodium, acétate de sodium, oléate de sodium, polyéthylèneglycol, acides aminés comme la leucine, triglycérides d'acides gras saturés d'origine végétale, par exemple diisostéarate de triglycérine, esters d'acides à longue chaîne, par exemple oléate d'oléyle. oleyloleate, Isopropylmyristate, Isopropylpalmitate, 2-Ethylhexyl-palmitate, Isooctylstearate, Isopropylstearate, lauric acid-hexylester, di-n-butyladipate, alkoholes à longue chaîne, par exemple hexylalcohol, octylalkohol, decylalkohol, oleylalkohol, 2-octyldodecanol, 2-hexyldecanol, 2-octyldecanol.

4. Médicament destiné à être utilisé dans le traitement des infections virales de l'homme et/ou des mammifères selon une ou plusieurs des revendications précédentes, **caractérisé en ce qu'**il contient la quantité efficace de silvestrol et/ou d'episilvestrol sous forme protonée ou déprotonée, c'est-à-dire sous forme de sel.

5. Médicament destiné à être utilisé dans le traitement des infections virales de l'homme et/ou des mammifères selon une ou plusieurs des revendications précédentes, **caractérisé en ce qu'**il est appliqué de manière préventive pour être utilisé dans le traitement de l'homme et/ou des mammifères.
